# EUROPEAN PATENT SPECIFICATION

(11) **EP 0 880 312 B1**
(45) Date of publication and mention of the grant of the patent: **08.03.2006**
(21) Application number: 96943782.1
(22) Date of filing: 13.12.1996
(51) Int. Cl.: A01H 1/06, A01H 5/10, A01H 1/00, C12N 15/00, C07C 57/02, C07C 57/03, C07C 53/126

(54) **PLANTS HAVING MUTANT SEQUENCES THAT CONFER ALTERED FATTY ACID PROFILES**
Pflanzen mit mutierten Sequenzen, welche ein veränderten Fettsäuregehalt vermitteln
VEGETAUX COMPORTANT DES SEQUENCES MUTANTES CONFERANT DES PROFILS D'ACIDES GRAS MODIFIES

(30) Priority: 14.12.1995 US 572027
(43) Date of publication of application: 02.12.1998
(60) Divisional application: 06075031.2
(73) Proprietor: Cargill Incorporated, Wayzata, Minnesota 55391 (US)
(72) Inventor: DeBONTE, R., Lorin, Fort Collins, CO 80525 (US); FAN, Zhegong, Fort Collins, CO 80525 (US)
(74) Representative: Fisher, Adrian John
(86) International application number: PCT/US1996/020090
(87) International publication number: WO 1997/021340

(56) References cited:
- EP-A- 0 323 753
- WO-A-91/15578
- WO-A-93/12245
- WO-A-94/11516
- WO-A-94/18337
- US-A- 4 948 811
- US-A- 5 387 758
- US-A- 5 434 283
- US-A- 5 668 299
- SHANKLIN ET AL: "Eight histidine residues are ctalyticaly essential in a membrane-associated iron enzyme, stearoyl-CoA desaturase, and are conserved in alkane hydroxylase and xylene monooxygenase" BIOCHEMISTRY, AMERICAN CHEMICAL SOCIETY. EASTON, PA, US, no. 33, 1994, pages 12787-12794, XP002075534 ISSN: 0006-2960
- IBA KOH ET AL: "A gene encoding a chloroplast omega-3 fatty acid desaturase complements alterations in fatty acid desaturation and chloroplast copy number of the fad7 mutant of Arabidopsis thaliana." JOURNAL OF BIOLOGICAL CHEMISTRY, vol. 268, no. 32, 1993, pages 24099-24105, XP001095029 ISSN: 0021-9258
- YADAV NARENDRA S ET AL: "Cloning of higher plant omega-3 fatty acid desaturases." PLANT PHYSIOLOGY (ROCKVILLE), vol. 103, no. 2, 1993, pages 467-476, XP001094712 ISSN: 0032-0889
- Z. PFLAZENZUCHTG., 1975, Vol. 73, ROBBELEN, "Genetical and Physiological Investigations on Mutants for Polyenoic Fatty Acids in Rapeseed, Brassica Napus L.", pages 93-105.
- SCIENCE, Vol. 268, 05 May 1995, TOPFER et al., "Modification of Plant Lipid Synthesis", pages 681-686.
- CAN. J. PLANT SCI., Apr. 1988, Vol. 68, SCARTH et al., "Stellar Low Linolenic -High Linoleic Acid Summer Rape", pages 509-511.

### SEQUENCE LISTING

(1) GENERAL INFORMATION:
   (i) APPLICANT: Cargill, Incorporated
   (ii) TITLE OF INVENTION: PLANTS HAVING MUTANT SEQUENCES THAT CONFER ALTERED FATTY ACID PROFILES
   (iii) NUMBER OF SEQUENCES: 8
   (iv) CORRESPONDENCE ADDRESS:
      (A) ADDRESSEE: Fish & Richardson, P.C., P.A.
      (B) STREET: 60 South Sixth Street, Suite 3300
      (C) CITY: Minneapolis
      (D) STATE: MN
      (E) COUNTRY: USA
      (F) ZIP: 55402
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30
   (vi) CURRENT APPLICATION DATA:
      (A) APPLICATION NUMBER: PCT/US96/
      (B) FILING DATE: 13-DEC-1996
      (C) CLASSIFICATION:
   (vii) PRIOR APPLICATION DATA:
      (A) APPLICATION NUMBER: US 08/572,027
      (B) FILING DATE: 14-DEC-1995
      (C) CLASSIFICATION:
   (viii) ATTORNEY/AGENT INFORMATION:
      (A) NAME: Ellinger, Mark S.
      (B) REGISTRATION NUMBER: 34,812
      (C) REFERENCE/DOCKET NUMBER: 07148/049WO1
   (ix) TELECOMMUNICATION INFORMATION:
      (A) TELEPHONE: 612/335-5070
      (B) TELEFAX: 612/288-9696
(2) INFORMATION FOR SEQ ID NO:1:
   (i) SEQUENCE CHARACTERISTICS:
      (A) LENGTH: 1155 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (A) ORGANISM: Brassica napus
   (ix) FEATURE:
      (D) OTHER INFORMATION: Wild type D form.
   (xi) SEQUENCE DESCRIPTION: SEQ ID NO:1:
   ATG GGT GCA GGT GGA AGA ATG CAA GTG TCT CCT CCC TCC AAG AAG TCT 48
   Met Gly Ala Gly Gly Arg Met Gln Val Ser Pro Pro Ser Lys Lys Ser
   1 5 10 15
   GAA ACC GAC ACC ATC AAG CGC GTA CCC TGC GAG ACA CCG CCC TTC ACT 96
   Glu Thr Asp Thr Ile Lys Arg Val Pro Cys Glu Thr Pro Pro Phe Thr
   20 25 30
   GTC GGA GAA CTC AAG AAA GCA ATC CCA CCG CAC TGT TTC AAA CGC TCG 144
   Val Gly Glu Leu Lys Lys Ala Ile Pro Pro His Cys Phe Lys Arg Ser
   35 40 45
   ATC CCT CGC TCT TTC TCC TAC CTC ATC TGG GAC ATC ATC ATA GCC TCC 192
   Ile Pro Arg Ser Phe Ser Tyr Leu Ile Trp Asp Ile Ile Ile Ala Ser
   50 55 60
   TGC TTC TAC TAC NTC GCC ACC ACT TAC TTC CCT CTC CTC CCT CAC CCT 240
   Cys Phe Tyr Tyr Xaa Ala Thr Thr Tyr Phe Pro Leu Leu Pro His Pro
   65 70 75 80
   CTC TCC TAC TTC GCC TGG CCT CTC TAC TGG GCC TGC CAA GGG TGC GTC 288
   Leu Ser Tyr Phe Ala Trp Pro Leu Tyr Trp Ala Cys Gln Gly Cys Val
   85 90 95
   CTA ACC GGC GTC TGG GTC ATA GCC CAC GAA TGC GGC CAC CAC GCC TTC 336
   Leu Thr Gly Val Trp Val Ile Ala His Glu Cys Gly His His Ala Phe
   100 105 110
   AGC GAC TAC CAG TGG CTT GAC GAC ACC GTC GGT CTC ATC TTC CAC TCC 384
   Ser Asp Tyr Gln Trp Leu Asp Asp Thr Val Gly Leu Ile Phe His Ser
   115 120 125
   TTC CTC CTC GTC CCT TAC TTC TCC TGG AAG TAC AGT CAT CGC AGC CAC 432
   Phe Leu Leu Val Pro Tyr Phe Ser Trp Lys Tyr Ser His Arg Ser His
   130 135 140
   CAT TCC AAC ACT GGC TCC CTC GAG AGA GAC GAA GTG TTT GTC CCC AAG 480
   His Ser Asn Thr Gly Ser Leu Glu Arg Asp Glu Val Phe Val Pro Lys
   145 150 155 160
   AAG AAG TCA GAC ATC AAG TGG TAC GGC AAG TAC CTC AAC AAC CCT TTG 528
   Lys Lys Ser Asp Ile Lys Trp Tyr Gly Lys Tyr Leu Asn Asn Pro Leu
   165 170 175
   GGA CGC ACC GTG ATG TTA ACG GTT CAG TTC ACT CTC GGC TGG CCG TTG 576
   Gly Arg Thr Val Met Leu Thr Val Gln Phe Thr Leu Gly Trp Pro Leu
   180 185 190
   TAC TTA GCC TTC AAC GTC TCG GGA AGA CCT TAC GAC GGC GGC TTC CGT 624
   Tyr Leu Ala Phe Asn Val Ser Gly Arg Pro Tyr Asp Gly Gly Phe Arg
   195 200 205
   TGC CAT TTC CAC CCC AAC GCT CCC ATC TAC AAC GAC CGC GAG CGT CTC 672
   Cys His Phe His Pro Asn Ala Pro Ile Tyr Asn Asp Arg Glu Arg Leu
   210 215 220
   CAG ATA TAC ATC TCC GAC GCT GGC ATC CTC GCC GTC TGC TAC GGT CTC 720
   Gln Ile Tyr Ile Ser Asp Ala Gly Ile Leu Ala Val Cys Tyr Gly Leu
   225 230 235 240
   TTC CGT TAC GCC GCC GGC CAG GGA GTG GCC TCG ATG GTC TGC TTC TAC 768
   Phe Arg Tyr Ala Ala Gly Gln Gly Val Ala Ser Met Val Cys Phe Tyr
   245 250 255
   GGA GTC CCG CTT CTG ATT GTC AAT GGT TTC CTC GTG TTG ATC ACT TAC 816
   Gly Val Pro Leu Leu Ile Val Asn Gly Phe Leu Val Leu Ile Thr Tyr
   260 265 270
   TTG CAG CAC ACG CAT CCT TCC CTG CCT CAC TAC GAT TCG TCC GAG TGG      864
   Leu Gln His Thr His Pro Ser Leu Pro His Tyr Asp Ser Ser Glu Trp
           275                 280                 285
   GAT TGG TTC AGG GGA GCT TTG GCT ACC GTT GAC AGA GAC TAC GGA ATC      912
   Asp Trp Phe Arg Gly Ala Leu Ala Thr Val Asp Arg Asp Tyr Gly Ile
       290                 295                 300
   TTG AAC AAG GTC TTC CAC AAT ATT ACC GAC ACG CAC GTG GCC CAT CAT      960
   Leu Asn Lys Val Phe His Asn Ile Thr Asp Thr His Val Ala His His
   305                 310                 315                 320
   CCG TTC TCC ACG ATG CCG CAT TAT CAC GCG ATG GAA GCT ACC AAG GCG     1008
   Pro Phe Ser Thr Met Pro His Tyr His Ala Met Glu Ala Thr Lys Ala
                   325                 330                 335
   ATA AAG CCG ATA CTG GGA GAG TAT TAT CAG TTC GAT GGG ACG CCG GTG     1056
   Ile Lys Pro Ile Leu Gly Glu Tyr Tyr Gln Phe Asp Gly Thr Pro Val
               340                 345                 350
   GTT AAG GCG ATG TGG AGG GAG GCG AAG GAG TGT ATC TAT GTG GAA CCG     1104
   Val Lys Ala Met Trp Arg Glu Ala Lys Glu Cys Ile Tyr Val Glu Pro
           355                 360                 365
   GAC AGG CAA GGT GAG AAG AAA GGT GTG TTC TGG TAC AAC AAT AAG TTA T   1153
   Asp Arg Gln Gly Glu Lys Lys Gly Val Phe Trp Tyr Asn Asn Lys Leu
       370                 375                 380
   GA                                                                  1155
(2) INFORMATION FOR SEQ ID NO:2:
   (i) SEQUENCE CHARACTERISTICS:
      (A) LENGTH: 384 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Gly Ala Gly Gly Arg Met Gln Val Ser Pro Pro Ser Lys Lys Ser
    1               5                  10                  15
   Glu Thr Asp Thr Ile Lys Arg Val Pro Cys Glu Thr Pro Pro Phe Thr
               20                  25                  30
   Val Gly Glu Leu Lys Lys Ala Ile Pro Pro His Cys Phe Lys Arg Ser
           35                  40                  45
   Ile Pro Arg Ser Phe Ser Tyr Leu Ile Trp Asp Ile Ile Ile Ala Ser
       50                  55                  60
   Cys Phe Tyr Tyr Xaa Ala Thr Thr Tyr Phe Pro Leu Leu Pro His Pro
   65                  70                  75                  80
   Leu Ser Tyr Phe Ala Trp Pro Leu Tyr Trp Ala Cys Gln Gly Cys Val
                   85                  90                  95
   Leu Thr Gly Val Trp Val Ile Ala His Glu Cys Gly His His Ala Phe
               100                 105                 110
   Ser Asp Tyr Gln Trp Leu Asp Asp Thr Val Gly Leu Ile Phe His Ser
           115                 120                 125
   Phe Leu Leu Val Pro Tyr Phe Ser Trp Lys Tyr Ser His Arg Ser His
       130                 135                 140
   His Ser Asn Thr Gly Ser Leu Glu Arg Asp Glu Val Phe Val Pro Lys
   145                 150                 155                 160
   Lys Lys Ser Asp Ile Lys Trp Tyr Gly Lys Tyr Leu Asn Asn Pro Leu
                   165                 170                 175
   Gly Arg Thr Val Met Leu Thr Val Gln Phe Thr Leu Gly Trp Pro Leu
               180                 185                 190
   Tyr Leu Ala Phe Asn Val Ser Gly Arg Pro Tyr Asp Gly Gly Phe Arg
           195                 200                 205
   Cys His Phe His Pro Asn Ala Pro Ile Tyr Asn Asp Arg Glu Arg Leu
       210                 215                 220
   Gln Ile Tyr Ile Ser Asp Ala Gly Ile Leu Ala Val Cys Tyr Gly Leu
   225                 230                 235                 240
   Phe Arg Tyr Ala Ala Gly Gln Gly Val Ala Ser Met Val Cys Phe Tyr
                   245                 250                 255
   Gly Val Pro Leu Leu Ile Val Asn Gly Phe Leu Val Leu Ile Thr Tyr
               260                 265                 270
   Leu Gln His Thr His Pro Ser Leu Pro His Tyr Asp Ser Ser Glu Trp
           275                 280                 285
   Asp Trp Phe Arg Gly Ala Leu Ala Thr Val Asp Arg Asp Tyr Gly Ile
       290                 295                 300
   Leu Asn Lys Val Phe His Asn Ile Thr Asp Thr His Val Ala His His
   305                 310                 315                 320
   Pro Phe Ser Thr Met Pro His Tyr His Ala Met Glu Ala Thr Lys Ala
                   325                 330                 335
   Ile Lys Pro Ile Leu Gly Glu Tyr Tyr Gln Phe Asp Gly Thr Pro Val
               340                 345                 350
   Val Lys Ala Met Trp Arg Glu Ala Lys Glu Cys Ile Tyr Val Glu Pro
           355                 360                 365
   Asp Arg Gln Gly Glu Lys Lys Gly Val Phe Trp Tyr Asn Asn Lys Leu
       370                 375                 380
(2) INFORMATION FOR SEQ ID NO:3:
   (i) SEQUENCE CHARACTERISTICS:
      (A) LENGTH: 1155 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (A) ORGANISM: Brassica napus
   (vii) IMMEDIATE SOURCE:
      (B) CLONE: IMC129
   (ix) FEATURE:
      (D) OTHER INFORMATION: G to A transversion mutation at nucleotide 316 of the D form.
   (xi) SEQUENCE DESCRIPTION: SEQ ID NO:3:
   ATG GGT GCA GGT GGA AGA ATG CAA GTG TCT CCT CCC TCC AAG AAG TCT 48
   Met Gly Ala Gly Gly Arg Met Gln Val Ser Pro Pro Ser Lys Lys Ser
   1 5 10 15
   GAA ACC GAC ACC ATC AAG CGC GTA CCC TGC GAG ACA CCG CCC TTC ACT 96
   Glu Thr Asp Thr Ile Lys Arg Val Pro Cys Glu Thr Pro Pro Phe Thr
   20 25 30
   GTC GGA GAA CTC AAG AAA GCA ATC CCA CCG CAC TGT TTC AAA CGC TCG 144
   Val Gly Glu Leu Lys Lys Ala Ile Pro Pro His Cys Phe Lys Arg Ser
   35 40 45
   ATC CCT CGC TCT TTC TCC TAC CTC ATC TGG GAC ATC ATC ATA GCC TCC 192
   Ile Pro Arg Ser Phe Ser Tyr Leu Ile Trp Asp Ile Ile Ile Ala Ser
   50 55 60
   TGC TTC TAC TAC NTC GCC ACC ACT TAC TTC CCT CTC CTC CCT CAC CCT 240
   Cys Phe Tyr Tyr Xaa Ala Thr Thr Tyr Phe Pro Leu Leu Pro His Pro
   65 70 75 80
   CTC TCC TAC TTC GCC TGG CCT CTC TAC TGG GCC TGC CAA GGG TGC GTC 288
   Leu Ser Tyr Phe Ala Trp Pro Leu Tyr Trp Ala Cys Gln Gly Cys Val
   85 90 95
   CTA ACC GGC GTC TGG GTC ATA GCC CAC AAG TGC GGC CAC CAC GCC TTC 336
   Leu Thr Gly Val Trp Val Ile Ala His Lys Cys Gly His His Ala Phe
   100 105 110
   AGC GAC TAC CAG TGG CTT GAC GAC ACC GTC GGT CTC ATC TTC CAC TCC 384
   Ser Asp Tyr Gln Trp Leu Asp Asp Thr Val Gly Leu Ile Phe His Ser
   115 120 125
   TTC CTC CTC GTC CCT TAC TTC TCC TGG AAG TAC AGT CAT CGC AGC CAC 432
   Phe Leu Leu Val Pro Tyr Phe Ser Trp Lys Tyr Ser His Arg Ser His
   130 135 140
   CAT TCC AAC ACT GGC TCC CTC GAG AGA GAC GAA GTG TTT GTC CCC AAG 480
   His Ser Asn Thr Gly Ser Leu Glu Arg Asp Glu Val Phe Val Pro Lys
   145 150 155 160
   AAG AAG TCA GAC ATC AAG TGG TAC GGC AAG TAC CTC AAC AAC CCT TTG 528
   Lys Lys Ser Asp Ile Lys Trp Tyr Gly Lys Tyr Leu Asn Asn Pro Leu
   165 170 175
   GGA CGC ACC GTG ATG TTA ACG GTT CAG TTC ACT CTC GGC TGG CCG TTG 576
   Gly Arg Thr Val Met Leu Thr Val Gln Phe Thr Leu Gly Trp Pro Leu
   180 185 190
   TAC TTA GCC TTC AAC GTC TCG GGA AGA CCT TAC GAC GGC GGC TTC CGT 624
   Tyr Leu Ala Phe Asn Val Ser Gly Arg Pro Tyr Asp Gly Gly Phe Arg
   195 200 205
   TGC CAT TTC CAC CCC AAC GCT CCC ATC TAC AAC GAC CGC GAG CGT CTC 672
   Cys His Phe His Pro Asn Ala Pro Ile Tyr Asn Asp Arg Glu Arg Leu
   210 215 220
   CAG ATA TAC ATC TCC GAC GCT GGC ATC CTC GCC GTC TGC TAC GGT CTC 720
   Gln Ile Tyr Ile Ser Asp Ala Gly Ile Leu Ala Val Cys Tyr Gly Leu
   225 230 235 240
   TTC CGT TAC GCC GCC GGC CAG GGA GTG GCC TCG ATG GTC TGC TTC TAC 768
   Phe Arg Tyr Ala Ala Gly Gln Gly Val Ala Ser Met Val Cys Phe Tyr
   245 250 255
   GGA GTC CCG CTT CTG ATT GTC AAT GGT TTC CTC GTG TTG ATC ACT TAC 816
   Gly Val Pro Leu Leu Ile Val Asn Gly Phe Leu Val Leu Ile Thr Tyr
   260 265 270
   TTG CAG CAC ACG CAT CCT TCC CTG CCT CAC TAC GAT TCG TCC GAG TGG      864
   Leu Gln His Thr His Pro Ser Leu Pro His Tyr Asp Ser Ser Glu Trp
           275                 280                     285
   GAT TGG TTC AGG GGA GCT TTG GCT ACC GTT GAC AGA GAC TAC GGA ATC      912
   Asp Trp Phe Arg Gly Ala Leu Ala Thr Val Asp Arg Asp Tyr Gly Ile
       290                 295                     300
   TTG AAC AAG GTC TTC CAC AAT ATT ACC GAC ACG CAC GTG GCC CAT CAT      960
   Leu Asn Lys Val Phe His Asn Ile Thr Asp Thr His Val Ala His His
   305                 310                     315                 320
   CCG TTC TCC ACG ATG CCG CAT TAT CAC GCG ATG GAA GCT ACC AAG GCG     1008
   Pro Phe Ser Thr Met Pro His Tyr His Ala Met Glu Ala Thr Lys Ala
                   325                     330                 335
   ATA AAG CCG ATA CTG GGA GAG TAT TAT CAG TTC GAT GGG ACG CCG GTG     1056
   Ile Lys Pro Ile Leu Gly Glu Tyr Tyr Gln Phe Asp Gly Thr Pro Val
               340                     345                     350
   GTT AAG GCG ATG TGG AGG GAG GCG AAG GAG TGT ATC TAT GTG GAA CCG     1104
   Val Lys Ala Met Trp Arg Glu Ala Lys Glu Cys Ile Tyr Val Glu Pro
           355                     360                     365
   GAC AGG CAA GGT GAG AAG AAA GGT GTG TTC TGG TAC AAC AAT AAG TTA T   1153
   Asp Arg Gln Gly Glu Lys Lys Gly Val Phe Trp Tyr Asn Asn Lys Leu
       370                     375                     380
   GA                                                                  1155
(2) INFORMATION FOR SEQ ID NO:4:
   (i) SEQUENCE CHARACTERISTICS:
      (A) LENGTH: 384 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Gly Ala Gly Gly Arg Met Gln Val Ser Pro Pro Ser Lys Lys Ser
    1               5                  10                  15
   Glu Thr Asp Thr Ile Lys Arg Val Pro Cys Glu Thr Pro Pro Phe Thr
               20                  25                  30
   Val Gly Glu Leu Lys Lys Ala Ile Pro Pro His Cys Phe Lys Arg Ser
           35                  40                  45
   Ile Pro Arg Ser Phe Ser Tyr Leu Ile Trp Asp Ile Ile Ile Ala Ser
       50                  55                  60
   Cys Phe Tyr Tyr Xaa Ala Thr Thr Tyr Phe Pro Leu Leu Pro His Pro
   65                  70                  75                  80
   Leu Ser Tyr Phe Ala Trp Pro Leu Tyr Trp Ala Cys Gln Gly Cys Val
                   85                  90                  95
   Leu Thr Gly Val Trp Val Ile Ala His Lys Cys Gly His His Ala Phe
               100                 105                 110
   Ser Asp Tyr Gln Trp Leu Asp Asp Thr Val Gly Leu Ile Phe His Ser
           115                 120                 125
   Phe Leu Leu Val Pro Tyr Phe Ser Trp Lys Tyr Ser His Arg Ser His
       130                 135                 140
   His Ser Asn Thr Gly Ser Leu Glu Arg Asp Glu Val Phe Val Pro Lys
   145 150 155 160
   Lys Lys Ser Asp Ile Lys Trp Tyr Gly Lys Tyr Leu Asn Asn Pro Leu
   165 170 175
   Gly Arg Thr Val Met Leu Thr Val Gln Phe Thr Leu Gly Trp Pro Leu
   180 185 190
   Tyr Leu Ala Phe Asn Val Ser Gly Arg Pro Tyr Asp Gly Gly Phe Arg
   195 200 205
   Cys His Phe His Pro Asn Ala Pro Ile Tyr Asn Asp Arg Glu Arg Leu
   210 215 220
   Gln Ile Tyr Ile Ser Asp Ala Gly Ile Leu Ala Val Cys Tyr Gly Leu
   225 230 235 240
   Phe Arg Tyr Ala Ala Gly Gln Gly Val Ala Ser Met Val Cys Phe Tyr
   245 250 255
   Gly Val Pro Leu Leu Ile Val Asn Gly Phe Leu Val Leu Ile Thr Tyr
   260 265 270
   Leu Gln His Thr His Pro Ser Leu Pro His Tyr Asp Ser Ser Glu Trp
   275 280 285
   Asp Trp Phe Arg Gly Ala Leu Ala Thr Val Asp Arg Asp Tyr Gly Ile
   290 295 300
   Leu Asn Lys Val Phe His Asn Ile Thr Asp Thr His Val Ala His His
   305 310 315 320
   Pro Phe Ser Thr Met Pro His Tyr His Ala Met Glu Ala Thr Lys Ala
   325 330 335
   Ile Lys Pro Ile Leu Gly Glu Tyr Tyr Gln Phe Asp Gly Thr Pro Val
   340 345 350
   Val Lys Ala Met Trp Arg Glu Ala Lys Glu Cys Ile Tyr Val Glu Pro
   355 360 365
   Asp Arg Gln Gly Glu Lys Lys Gly Val Phe Trp Tyr Asn Asn Lys Leu
   370 375 380
(2) INFORMATION FOR SEQ ID NO:5:
   (i) SEQUENCE CHARACTERISTICS:
      (A) LENGTH: 1155 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (A) ORGANISM: Brassica napus
   (ix) FEATURE:
      (D) OTHER INFORMATION: Wild type F form.
   (xi) SEQUENCE DESCRIPTION: SEQ ID NO:5:
   ATG GGT GCA GGT GGA AGA ATG CAA GTG TCT CCT CCC TCC AAA AAG TCT      48
   Met Gly Ala Gly Gly Arg Met Gln Val Ser Pro Pro Ser Lys Lys Ser
     1               5                  10                  15
   GAA ACC GAC AAC ATC AAG CGC GTA CCC TGC GAG ACA CCG CCC TTC ACT      96
   Glu Thr Asp Asn Ile Lys Arg Val Pro Cys Glu Thr Pro Pro Phe Thr
                20                  25                  30
   GTC GGA GAA CTC AAG AAA GCA ATC CCA CCG CAC TGT TTC AAA CGC TCG     144
   Val Gly Glu Leu Lys Lys Ala Ile Pro Pro His Cys Phe Lys Arg Ser
            35                  40                  45
   ATC CCT CGC TCT TTC TCC TAC CTC ATC TGG GAC ATC ATC ATA GCC TCC     192
   Ile Pro Arg Ser Phe Ser Tyr Leu Ile Trp Asp Ile Ile Ile Ala Ser
        50                  55                  60
   TGC TTC TAC TAC GTC GCC ACC ACT TAC TTC CCT CTC CTC CCT CAC CCT     240
   Cys Phe Tyr Tyr Val Ala Thr Thr Tyr Phe Pro Leu Leu Pro His Pro
    65                  70                  75                  80
   CTC TCC TAC TTC GCC TGG CCT CTC TAC TGG GCC TGC CAG GGC TGC GTC     288
   Leu Ser Tyr Phe Ala Trp Pro Leu Tyr Trp Ala Cys Gln Gly Cys Val
                    85                  90                  95
   CTA ACC GGC GTC TGG GTC ATA GCC CAC GAG TGC GGC CAC CAC GCC TTC     336
   Leu Thr Gly Val Trp Val Ile Ala His Glu Cys Gly His His Ala Phe
               100                 105                 110
   AGC GAC TAC CAG TGG CTG GAC GAC ACC GTC GGC CTC ATC TTC CAC TCC     384
   Ser Asp Tyr Gln Trp Leu Asp Asp Thr Val Gly Leu Ile Phe His Ser
           115                 120                 125
   TTC CTC CTC GTC CCT TAC TTC TCC TGG AAG TAC AGT CAT CGA CGC CAC     432
   Phe Leu Leu Val Pro Tyr Phe Ser Trp Lys Tyr Ser His Arg Arg His
       130                 135                 140
   CAT TCC AAC ACT GGC TCC CTC GAG AGA GAC GAA GTG TTT GTC CCC AAG     480
   His Ser Asn Thr Gly Ser Leu Glu Arg Asp Glu Val Phe Val Pro Lys
   145                 150                 155                 160
   AAG AAG TCA GAC ATC AAG TGG TAC GGC AAG TAC CTC AAC AAC CCT TTG     528
   Lys Lys Ser Asp Ile Lys Trp Tyr Gly Lys Tyr Leu Asn Asn Pro Leu
                   165                 170                 175
   GGA CGC ACC GTG ATG TTA ACG GTT CAG TTC ACT CTC GGC TGG CCT TTG     576
   Gly Arg Thr Val Met Leu Thr Val Gln Phe Thr Leu Gly Trp Pro Leu
               180                 185                 190
   TAC TTA GCC TTC AAC GTC TCG GGG AGA CCT TAC GAC GGC GGC TTC GCT     624
   Tyr Leu Ala Phe Asn Val Ser Gly Arg Pro Tyr Asp Gly Gly Phe Ala
           195                 200                 205
   TGC CAT TTC CAC CCC AAC GCT CCC ATC TAC AAC GAC CGC GAG CGT CTC     672
   Cys His Phe His Pro Asn Ala Pro Ile Tyr Asn Asp Arg Glu Arg Leu
       210                 215                 220
   CAG ATA TAC ATC TCC GAC GCT GGC ATC CTC GCC GTC TGC TAC GGT CTC     720
   Gln Ile Tyr Ile Ser Asp Ala Gly Ile Leu Ala Val Cys Tyr Gly Leu
   225                 230                 235                 240
   TAC CGC TAC GCT GCT GTC CAA GGA GTT GCC TCG ATG GTC TGC TTC TAC     768
   Tyr Arg Tyr Ala Ala Val Gln Gly Val Ala Ser Met Val Cys Phe Tyr
                   245                 250                 255
   GGA GTT CCG CTT CTG ATT GTC AAT GGG TTC TTA GTT TTG ATC ACT TAC     816
   Gly Val Pro Leu Leu Ile Val Asn Gly Phe Leu Val Leu Ile Thr Tyr
               260                 265                 270
   TTG CAG CAC ACG CAT CCT TCC CTG CCT CAC TAT GAC TCG TCT GAG TGG        864
   Leu Gln His Thr His Pro Ser Leu Pro His Tyr Asp Ser Ser Glu Trp
           275                 280                     285
   GAT TGG TTG AGG GGA GCT TTG GCC ACC GTT GAC AGA GAC TAC GGA ATC        912
   Asp Trp Leu Arg Gly Ala Leu Ala Thr Val Asp Arg Asp Tyr Gly Ile
       290                 295                     300
   TTG AAC AAG GTC TTC CAC AAT ATC ACG GAC ACG CAC GTG GCG CAT CAC        960
   Leu Asn Lys Val Phe His Asn Ile Thr Asp Thr His Val Ala His His
   305                 310                     315                 320
   CTG TTC TCG ACC ATG CCG CAT TAT CAT GCG ATG GAA GCT ACG AAG GCG       1008
   Leu Phe Ser Thr Met Pro His Tyr His Ala Met Glu Ala Thr Lys Ala
                   325                     330                 335
   ATA AAG CCG ATA CTG GGA GAG TAT TAT CAG TTG CAT GGG ACG CCG GTG       1056
   Ile Lys Pro Ile Leu Gly Glu Tyr Tyr Gln Leu His Gly Thr Pro Val
               340                 345                     350
   GTT AAG GCG ATG TGG AGG GAG GCG AAG GAG TGT ATC TAT GTG GAA CCG       1104
   Val Lys Ala Met Trp Arg Glu Ala Lys Glu Cys Ile Tyr Val Glu Pro
           355                 360                     365
   GAC AGG CAA GGT GAG AAG AAA GGT GTG TTC TGG TAC AAC AAT AAG TTA T     1153
   Asp Arg Gln Gly Glu Lys Lys Gly Val Phe Trp Tyr Asn Asn Lys Leu
       370                 375                     380
   GA                                                                    1155
(2) INFORMATION FOR SEQ ID NO:6:
   (i) SEQUENCE CHARACTERISTICS:
      (A) LENGTH: 384 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Gly Ala Gly Gly Arg Met Gln Val Ser Pro Pro Ser Lys Lys Ser
    1               5                  10                  15
   Glu Thr Asp Asn Ile Lys Arg Val Pro Cys Glu Thr Pro Pro Phe Thr
               20                  25                  30
   Val Gly Glu Leu Lys Lys Ala Ile Pro Pro His Cys Phe Lys Arg Ser
           35                  40                  45
   Ile Pro Arg Ser Phe Ser Tyr Leu Ile Trp Asp Ile Ile Ile Ala Ser
       50                  55                  60
   Cys Phe Tyr Tyr Val Ala Thr Thr Tyr Phe Pro Leu Leu Pro His Pro
   65                  70                  75                  80
   Leu Ser Tyr Phe Ala Trp Pro Leu Tyr Trp Ala Cys Gln Gly Cys Val
                   85                  90                  95
   Leu Thr Gly Val Trp Val Ile Ala His Glu Cys Gly His His Ala Phe
               100                 105                 110
   Ser Asp Tyr Gln Trp Leu Asp Asp Thr Val Gly Leu Ile Phe His Ser
           115                 120                 125
   Phe Leu Leu Val Pro Tyr Phe Ser Trp Lys Tyr Ser His Arg Arg His
       130                 135                 140
   His Ser Asn Thr Gly Ser Leu Glu Arg Asp Glu Val Phe Val Pro Lys
   145                 150                 155                 160
   Lys Lys Ser Asp Ile Lys Trp Tyr Gly Lys Tyr Leu Asn Asn Pro Leu
                   165                 170                 175
   Gly Arg Thr Val Met Leu Thr Val Gln Phe Thr Leu Gly Trp Pro Leu
               180                 185                 190
   Tyr Leu Ala Phe Asn Val Ser Gly Arg Pro Tyr Asp Gly Gly Phe Ala
           195                 200                 205
   Cys His Phe His Pro Asn Ala Pro Ile Tyr Asn Asp Arg Glu Arg Leu
       210                 215                 220
   Gln Ile Tyr Ile Ser Asp Ala Gly Ile Leu Ala Val Cys Tyr Gly Leu
   225                 230                 235                 240
   Tyr Arg Tyr Ala Ala Val Gln Gly Val Ala Ser Met Val Cys Phe Tyr
                   245                 250                 255
   Gly Val Pro Leu Leu Ile Val Asn Gly Phe Leu Val Leu Ile Thr Tyr
               260                 265                 270
   Leu Gln His Thr His Pro Ser Leu Pro His Tyr Asp Ser Ser Glu Trp
           275                 280                 285
   Asp Trp Leu Arg Gly Ala Leu Ala Thr Val Asp Arg Asp Tyr Gly Ile
       290                 295                 300
   Leu Asn Lys Val Phe His Asn Ile Thr Asp Thr His Val Ala His His
   305                 310                 315                 320
   Leu Phe Ser Thr Met Pro His Tyr His Ala Met Glu Ala Thr Lys Ala
                   325                 330                 335
   Ile Lys Pro Ile Leu Gly Glu Tyr Tyr Gln Leu His Gly Thr Pro Val
               340                 345                 350
   Val Lys Ala Met Trp Arg Glu Ala Lys Glu Cys Ile Tyr Val Glu Pro
           355                 360                 365
   Asp Arg Gln Gly Glu Lys Lys Gly Val Phe Trp Tyr Asn Asn Lys Leu
       370                 375                 380
(2) INFORMATION FOR SEQ ID NO:7:
   (i) SEQUENCE CHARACTERISTICS:
      (A) LENGTH: 1155 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTI-SENSE: NO
   (vi) ORIGINAL SOURCE:
      (A) ORGANISM: Brassica napus
   (vii) IMMEDIATE SOURCE:
      (B) CLONE: Q508
   (ix) FEATURE:
      (D) OTHER INFORMATION: T to A transversion mutation at nucleotide 515 of the F form.
   (xi) SEQUENCE DESCRIPTION: SEQ ID NO:7:
   ATG GGT GCA GGT GGA AGA ATG CAA GTG TCT CCT CCC TCC AAA AAG TCT      48
   Met Gly Ala Gly Gly Arg Met Gln Val Ser Pro Pro Ser Lys Lys Ser
    1               5                  10                  15
   GAA ACC GAC AAC ATC AAG CGC GTA CCC TGC GAG ACA CCG CCC TTC ACT      96
   Glu Thr Asp Asn Ile Lys Arg Val Pro Cys Glu Thr Pro Pro Phe Thr
               20                  25                  30
   GTC GGA GAA CTC AAG AAA GCA ATC CCA CCG CAC TGT TTC AAA CGC TCG     144
   Val Gly Glu Leu Lys Lys Ala Ile Pro Pro His Cys Phe Lys Arg Ser
           35                  40                  45
   ATC CCT CGC TCT TTC TCC TAC CTC ATC TGG GAC ATC ATC ATA GCC TCC     192
   Ile Pro Arg Ser Phe Ser Tyr Leu Ile Trp Asp Ile Ile Ile Ala Ser
       50                  55                  60
   TGC TTC TAC TAC GTC GCC ACC ACT TAC TTC CCT CTC CTC CCT CAC CCT     240
   Cys Phe Tyr Tyr Val Ala Thr Thr Tyr Phe Pro Leu Leu Pro His Pro
   65                  70                  75                  80
   CTC TCC TAC TTC GCC TGG CCT CTC TAC TGG GCC TGC CAG GGC TGC GTC     288
   Leu Ser Tyr Phe Ala Trp Pro Leu Tyr Trp Ala Cys Gln Gly Cys Val
                   85                  90                  95
   CTA ACC GGC GTC TGG GTC ATA GCC CAC GAG TGC GGC CAC CAC GCC TTC     336
   Leu Thr Gly Val Trp Val Ile Ala His Glu Cys Gly His His Ala Phe
               100                 105                 110
   AGC GAC TAC CAG TGG CTG GAC GAC ACC GTC GGC CTC ATC TTC CAC TCC     384
   Ser Asp Tyr Gln Trp Leu Asp Asp Thr Val Gly Leu Ile Phe His Ser
           115                 120                 125
   TTC CTC CTC GTC CCT TAC TTC TCC TGG AAG TAC AGT CAT CGA CGC CAC     432
   Phe Leu Leu Val Pro Tyr Phe Ser Trp Lys Tyr Ser His Arg Arg His
       130                 135                 140
   CAT TCC AAC ACT GGC TCC CTC GAG AGA GAC GAA GTG TTT GTC CCC AAG     480
   His Ser Asn Thr Gly Ser Leu Glu Arg Asp Glu Val Phe Val Pro Lys
   145                 150                 155                 160
   AAG AAG TCA GAC ATC AAG TGG TAC GGC AAG TAC CAC AAC AAC CCT TTG     528
   Lys Lys Ser Asp Ile Lys Trp Tyr Gly Lys Tyr His Asn Asn Pro Leu
                   165                 170                 175
   GGA CGC ACC GTG ATG TTA ACG GTT CAG TTC ACT CTC GGC TGG CCT TTG     576
   Gly Arg Thr Val Met Leu Thr Val Gln Phe Thr Leu Gly Trp Pro Leu
               180                 185                 190
   TAC TTA GCC TTC AAC GTC TCG GGG AGA CCT TAC GAC GGC GGC TTC GCT     624
   Tyr Leu Ala Phe Asn Val Ser Gly Arg Pro Tyr Asp Gly Gly Phe Ala
           195                 200                 205
   TGC CAT TTC CAC CCC AAC GCT CCC ATC TAC AAC GAC CGC GAG CGT CTC     672
   Cys His Phe His Pro Asn Ala Pro Ile Tyr Asn Asp Arg Glu Arg Leu
       210                 215                 220
   CAG ATA TAC ATC TCC GAC GCT GGC ATC CTC GCC GTC TGC TAC GGT CTC     720
   Gln Ile Tyr Ile Ser Asp Ala Gly Ile Leu Ala Val Cys Tyr Gly Leu
   225                 230                 235                 240
   TAC CGC TAC GCT GCT GTC CAA GGA GTT GCC TCG ATG GTC TGC TTC TAC     768
   Tyr Arg Tyr Ala Ala Val Gln Gly Val Ala Ser Met Val Cys Phe Tyr
                   245                 250                 255
   GGA GTT CCG CTT CTG ATT GTC AAT GGG TTC TTA GTT TTG ATC ACT TAC     816
   Gly Val Pro Leu Leu Ile Val Asn Gly Phe Leu Val Leu Ile Thr Tyr
               260                 265                 270
   TTG CAG CAC ACG CAT CCT TCC CTG CCT CAC TAT GAC TCG TCT GAG TGG      864
   Leu Gln His Thr His Pro Ser Leu Pro His Tyr Asp Ser Ser Glu Trp
           275                 280                 285
   GAT TGG TTG AGG GGA GCT TTG GCC ACC GTT GAC AGA GAC TAC GGA ATC      912
   Asp Trp Leu Arg Gly Ala Leu Ala Thr Val Asp Arg Asp Tyr Gly Ile
       290                 295                 300
   TTG AAC AAG GTC TTC CAC AAT ATC ACG GAC ACG CAC GTG GCG CAT CAC      960
   Leu Asn Lys Val Phe His Asn Ile Thr Asp Thr His Val Ala His His
   305                 310                 315                 320
   CTG TTC TCG ACC ATG CCG CAT TAT CAT GCG ATG GAA GCT ACG AAG GCG     1008
   Leu Phe Ser Thr Met Pro His Tyr His Ala Met Glu Ala Thr Lys Ala
                   325                 330                 335
   ATA AAG CCG ATA CTG GGA GAG TAT TAT CAG TTG CAT GGG ACG CCG GTG     1056
   Ile Lys Pro Ile Leu Gly Glu Tyr Tyr Gln Leu His Gly Thr Pro Val
               340                 345                 350
   GTT AAG GCG ATG TGG AGG GAG GCG AAG GAG TGT ATC TAT GTG GAA CCG     1104
   Val Lys Ala Met Trp Arg Glu Ala Lys Glu Cys Ile Tyr Val Glu Pro
           355                 360                 365
   GAC AGG CAA GGT GAG AAG AAA GGT GTG TTC TGG TAC AAC AAT AAG TTA T   1153
   Asp Arg Gln Gly Glu Lys Lys Gly Val Phe Trp Tyr Asn Asn Lys Leu
       370                 375                 380
   GA                                                                  1155
(2) INFORMATION FOR SEQ ID NO:8:
   (i) SEQUENCE CHARACTERISTICS:
      (A) LENGTH: 384 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Gly Ala Gly Gly Arg Met Gln Val Ser Pro Pro Ser Lys Lys Ser
   1               5                   10                  15
   Glu Thr Asp Asn Ile Lys Arg Val Pro Cys Glu Thr Pro Pro Phe Thr
               20                  25                  30
   Val Gly Glu Leu Lys Lys Ala Ile Pro Pro His Cys Phe Lys Arg Ser
           35                  40                  45
   Ile Pro Arg Ser Phe Ser Tyr Leu Ile Trp Asp Ile Ile Ile Ala Ser
       50                  55                  60
   Cys Phe Tyr Tyr Val Ala Thr Thr Tyr Phe Pro Leu Leu Pro His Pro
   65                  70                  75                  80
   Leu Ser Tyr Phe Ala Trp Pro Leu Tyr Trp Ala Cys Gln Gly Cys Val
                   85                  90                  95
   Leu Thr Gly Val Trp Val Ile Ala His Glu Cys Gly His His Ala Phe
               100                 105                 110
   Ser Asp Tyr Gln Trp Leu Asp Asp Thr Val Gly Leu Ile Phe His Ser
           115                 120                 125
   Phe Leu Leu Val Pro Tyr Phe Ser Trp Lys Tyr Ser His Arg Arg His
       130                 135                 140
   His Ser Asn Thr Gly Ser Leu Glu Arg Asp Glu Val Phe Val Pro Lys
   145 150 155 160
   Lys Lys Ser Asp Ile Lys Trp Tyr Gly Lys Tyr His Asn Asn Pro Leu
   165 170 175
   Gly Arg Thr Val Met Leu Thr Val Gln Phe Thr Leu Gly Trp Pro Leu
   180 185 190
   Tyr Leu Ala Phe Asn Val Ser Gly Arg Pro Tyr Asp Gly Gly Phe Ala
   195 200 205
   Cys His Phe His Pro Asn Ala Pro Ile Tyr Asn Asp Arg Glu Arg Leu
   210 215 220
   Gln Ile Tyr Ile Ser Asp Ala Gly Ile Leu Ala Val Cys Tyr Gly Leu
   225 230 235 240
   Tyr Arg Tyr Ala Ala Val Gln Gly Val Ala Ser Met Val Cys Phe Tyr
   245 250 255
   Gly Val Pro Leu Leu Ile Val Asn Gly Phe Leu Val Leu Ile Thr Tyr
   260 265 270
   Leu Gln His Thr His Pro Ser Leu Pro His Tyr Asp Ser Ser Glu Trp
   275 280 285
   Asp Trp Leu Arg Gly Ala Leu Ala Thr Val Asp Arg Asp Tyr Gly Ile
   290 295 300
   Leu Asn Lys Val Phe His Asn Ile Thr Asp Thr His Val Ala His His
   305 310 315 320
   Leu Phe Ser Thr Met Pro His Tyr His Ala Met Glu Ala Thr Lys Ala
   325 330 335
   Ile Lys Pro Ile Leu Gly Glu Tyr Tyr Gln Leu His Gly Thr Pro Val
   340 345 350
   Val Lys Ala Met Trp Arg Glu Ala Lys Glu Cys Ile Tyr Val Glu Pro
   355 360 365
   Asp Arg Gln Gly Glu Lys Lys Gly Val Phe Trp Tyr Asn Asn Lys Leu
   370 375 380

## Description

### Technical Field

This invention relates to *Brassica* seeds and plants having mutant sequences which confer altered fatty acid profiles on the seed oil. More particularly, the invention relates to mutant delta-12 and delta-15 fatty acid desaturase sequences in such plants which confer such profiles.

### Background of the Invention

Diets high in saturated fats increase low density lipoproteins (LDL) which mediate the deposition of cholesterol on blood vessels. High plasma levels of serum cholesterol are closely correlated with atherosclerosis and coronary heart disease (Conner et al., Coronary Heart Disease: Prevention, Complications, and Treatment, pp. 43-64, 1985). By producing oilseed *Brassica* varieties with reduced levels of individual and total saturated fats in the seed oil, oil-based food products which contain less saturated fats can be produced. Such products will benefit public health by reducing the incidence of atherosclerosis and coronary heart disease.

The dietary effects of monounsaturated fats have also been shown to have dramatic effects on health. Oleic acid, the only monounsaturated fat in most edible vegetable oils, lowers LDL as effectively as linoleic acid, but does not affect high density lipoproteins (HDL) levels (Mattson, F.H., J. Am. Diet. Assoc., 89:387-391, 1989; Mensink et al., New England J. Med., 321:436-441, 1989). Oleic acid is at least as effective in lowering plasma cholesterol as a diet low in fat and high in carbohydrates (Grundy, S.M., New England J. Med., 314:745-748, 1986; Mensink et al., New England J. Med., 321:436-441, 1989). In fact, a high oleic acid diet is preferable to low fat, high carbohydrate diets for diabetics (Garg et al., New England J. Med., 319:829-834, 1988). Diets high in monounsaturated fats are also correlated with reduced systolic blood pressure (Williams et al., J. Am. Med. Assoc., 257:3251-3256, 1987). Epidemiological studies have demonstrated that the "Mediterranean" diet, which is high in fat and monounsaturates, is not associated with coronary heart disease (Keys, A., Circulation, 44 (Suppl):1, 1970).

Many breeding studies have been conducted to improve the fatty acid profile of *Brassica* varieties. Pleines and Freidt, Fat Sci. Technol., 90(5), 167-171 (1988) describe plant lines with reduced C_{18:3} levels (2.5-5.8%) combined with high oleic content (73-79%). Rakow and McGregor, J. Amer. Oil Chem. Soc., 50, 400-403 (Oct. 1973) discuss problems associated with selecting mutants for linoleic and linolenic acids. In. Can. J. Plant Sci., 68, 509-511 (Apr. 1988) Stellar summer rape producing seed oil with 3% linolenic acid and 28% linoleic acid is disclosed. Roy and Tarr, Z. Pflanzenzuchtg, 95(3), 201-209 (1985) teaches transfer of genes through an interspecific cross from *Brassica juncea* into *Brassica napus* resulting in a reconstituted line combining high linoleic with low linolenic acid content. Roy and Tarr, Plant Breeding, 98, 89-96 (1987) discuss prospects for development of *B. napus L.* having improved linolenic and linolenic acid content. European Patent application 323,751 published July 12, 1989 discloses seeds and oils having greater than 79% oleic acid combined with less than 3.5% linolenic acid. Canvin, Can. J. Botany, 43, 63-69 (1965) discusses the effect of temperature on the fatty acid composition of oils from several seed crops including rapeseed.

Mutations typically are induced with extremely high doses of radiation and/or chemical mutagens (Gaul, H. Radiation Botany (1964) 4:155-232). High dose levels which exceed LD50, and typically reach LD90, led to maximum achievable mutation rates. In mutation breeding of *Brassica* varieties high levels of chemical mutagens alone or combined with radiation have induced a limited number of fatty acid mutations (Rakow, G.Z. Pflanzenzuchtg (1973) 69:62-82). The low α-linolenic acid mutation derived from the Rakow mutation breeding program did not have direct commercial application because of low seed yield. The first commercial cultivar using the low α-linolenic acid mutation derived in 1973 was released in 1988 as the variety Stellar (Scarth, R. et al., Can. J. Plant Sci. (1988) 68:509-511). Stellar was 20% lower yielding than commercial cultivars at the time of its release.

Canola-quality oilseed *Brassica* varieties with reduced levels of saturated fatty acids in the seed oil could be used to produce food products which promote cardiovascular health. Canola lines which are individually low in palmitic and stearic acid content or low in combination will reduce the levels of saturated fatty acids. Similarly, *Brassica* varieties with increased monounsaturate levels in the seed oil, and products derived from such oil, would improve lipid nutrition. Canola lines which are low in linoleic acid tend to have high oleic acid content, and can be used in the development of varieties having even higher oleic acid content.

Increased palmitic acid content provides a functional improvement in food applications. Oils high in palmitic acid content are particularly useful in the formulation of margarines. Thus, there is a need for manufacturing purposes for oils high in palmitic acid content.

Decreased α-linolenic acid content provides a functional improvement in food applications. Oils which are low in linolenic acid have increased stability. The rate of oxidation of lipid fatty acids increases with higher levels of linolenic acid leading to off-flavors and off-odors in foods. There is a need in the food industry for oils low in alpha linolenic acid.

Delta-12 fatty acid desaturase (also known as oleic desaturase) is involved in the enzymatic conversion of oleic acid to linoleic acid. Delta-15 fatty acid desaturase (also known as linoleic acid desaturase) is involved in the enzymatic conversion of linoleic acid to α-linolenic acid. A microsomal delta-12 desaturase has been cloned and characterized using T-DNA tagging. Okuley, et al., Plant Cell 6:147-158 (1994). The nucleotide sequences of higher plant genes encoding microsomal delta-12 fatty acid desaturase are described in Lightner et al., WO94/11516. Sequences of higher plant genes encoding microsomal and plastid delta-15 fatty acid desaturases are disclosed in Yadav, N., et al., Plant Physiol., 103:467-476 (1993), WO 93/11245 and Arondel, V. et al., Science, 258:1353-1355 (1992). However, there are no teachings that disclose mutations in delta-12 or delta-15 fatty acid desaturase coding sequences from plants. Furthermore, no methods have been described for developing plant lines that contain delta-12 or delta-15 fatty acid desaturase gene sequence mutations effective for altering the fatty acid composition of seeds.

### Summary of the Invention

The invention comprises *Brassicaceae* or *Helianthus* seeds, plants and plant lines having at least one mutation that controls the levels of unsaturated fatty acids in plants. One embodiment of the invention, as defined in claim 1, is an isolated nucleic acid fragment comprising a nucleotide sequence encoding a mutant delta-12 fatty acid desaturase conferring altered fatty composition in seeds when the fragment is present in a plant. A preferred sequence comprises a mutant sequence as shown in SEQ ID NO:3. Another embodiment of the invention, as defined in claim 8, is an isolated nucleic acid fragment comprising a nucleotide sequence encoding a mutant delta-15 fatty acid desaturase. A plant in this embodiment may be soybean, oilseed *Brassica* species, sunflower, castor bean or corn. The mutant sequence may be derived from, for example, a *Brassica napus, Brassica rapa, Brassica juncea* or *Helianthus* delta-12 or delta-15 gene.

Another embodiment of the invention as defined in claims 14 and 22, involves a method of producing a *Brassicaceae* or *Helianthus* plant line comprising the steps of: (a) inducing mutagenesis in cells of a starting variety of a *Brassicaceae* or *Helianthus* species; (b) obtaining progeny plants from the mutagenized cells; (c) identifying progeny plants that contain a mutation in a delta-12 or delta-15 fatty acid desaturase gene; and (d) producing a plant line by self or cross-pollination.

Yet another embodiment of the invention involves a method of producing plant lines containing altered levels of unsaturated fatty acids comprising: (a) crossing a first plant with a second plant having a mutant delta-12 or delta-15 fatty acid desaturase; (b) obtaining seeds from the cross of step (a); (c) growing fertile plants from such seeds; (d) obtaining progeny seed the plants of

### Brief Description of the Figures

Figure 1 is a histogram showing the frequency distribution of seed oil oleic acid (C_{18:1}) content in a segregating population of a Q508 X Westar cross. The bar labeled WSGA 1A represents the C_{18:1} content of the Westar parent. The bar labeled Q508 represents the C_{18:3} content of the Q508 parent.

### Description of the Preferred Embodiments

The U.S. Food and Drug Administration defines saturated fatty acids as the sum of lauric (C_{12:0}), myristic (C_{14:6}), palmitic (C_{16:0}) and stearic (C_{18:0}) acids. The term "FDA saturates" as used herein means this above-defined sum. Unless total saturate content is specified, the saturated fatty acid values expressed here include only "FDA saturates."

All percent fatty acids herein are percent by weight of the oil of which the fatty acid is a component.

As used herein, a "line" is a group of plants that display little or no genetic variation between individuals for at least one trait. Such lines may be created by several generations of self-pollination and selection, or vegetative propagation from a single parent using tissue or cell culture techniques. As used herein, the term "variety" refers to a line which is used for commercial production.

The term "mutagenesis" refers to the use of a mutagenic agent to induce random genetic mutations within a population of individuals. The treated population, or a subsequent generation of that population, is then screened for usable trait(s) that result from the mutations. A "population" is any group of individuals that share a common gene pool. As used herein "M₀" is untreated seed. As used herein, "M₁" is the seed (and resulting plants) exposed to a mutagenic agent, while "M₂" is the progeny (seeds and plants) of self-pollinated M₁ plants, "M₃" is the progeny of self-pollinated M₂ plants, and "M₄" is the progeny of self-pollinated M₃ plants. "M₅" is the progeny of self-pollinated M₄ plants. "M₆", "M₇", etc. are each the progeny of self-pollinated plants of the previous generation. The term "selfed" as used herein means self-pollinated.

"Stability" or "stable" as used herein means that with respect to a given fatty acid component, the component is maintained from generation to generation for at least two generations and preferably at least three generations at substantially the same level, e.g., preferably ±5%. The method of invention is capable of creating lines with improved fatty acid compositions stable up to ±5% from generation to generation. The above stability may be affected by temperature, location, stress and time of planting. Thus, comparison of fatty acid profiles should be made from seeds produced under similar growing conditions. Stability may be measured based on knowledge of prior generation.

Intensive breeding has produced Brassica plants whose seed oil contains less than 2% erucic acid. The same varieties have also been bred so that the defatted meal contains less than 30 µmol glucosinolates/gram. "Canola" as used herein refers to plant variety seed or oil which contains less than 2% erucic acid (C_{22:1}), and meal with less than 30 µmol glucosinolates/gram.

Applicants have discovered plants with mutations in a delta-12 fatty acid desaturase gene. Such plants have useful alterations in the fatty acid compositions of the seed oil. Such mutations confer, for example, an elevated oleic acid content, a decreased, stabilized linoleic acid content, or both elevated oleic acid and decreased, stabilized linoleic acid content.

Applicants have further discovered plants with mutations in a delta-15 fatty acid desaturase gene. Such plants have useful alterations in the fatty acid composition of the seed oil, e.g., a decreased, stabilized level of α-linolenic acid.

Applicants have further discovered isolated nucleic acid fragments comprising sequences that carry mutations within the coding sequence of delta-12 or delta-15 desaturases. The mutations confer desirable alterations in fatty acid levels in the seed oil of plants carrying such mutations. Delta-12 fatty acid desaturase is also known as omega-6 fatty acid desaturase and is sometimes referred to herein as 12-DES. Delta-15 fatty acid debaturase is also known omega-3 fatty acid as desaturase and is sometimes referred to herein as 15-DES.

A nucleic acid fragment of the invention contains a mutation in a microsomal delta-12 fatty acid desaturase coding sequence or in a microsomal delta-15 fatty acid desaturase coding sequence. Such a mutation renders the resulting desaturase gene product non-functional in plants, relative to the function of the gene product encoded by the wild-type sequence. The non-functionality of the 12-DES gene product can be inferred from the decreased level of reaction product (linoleic acid) and increased level of substrate (oleic acid) in plant tissues expressing the mutant sequence, compared to the corresponding levels in plant tissues expressing the wild-type sequence. The non-functionality of the 15-DES gene product can be inferred from the decreased level of reaction product (α-linolenic acid) and the increased level of substrate (linoleic acid) in plant tissues expressing the mutant sequence, compared to the corresponding levels in plant tissues expressing the wild-type sequence.

A nucleic acid fragment of the invention may comprise a portion of the coding sequence, e.g., at least about 10 nucleotides, provided that the fragment contains at least one mutation in the coding sequence. The length of a desired fragment depends upon the purpose for which the fragment will be used, e.g., PCR primer, site-directed mutagenesis and the like. In one embodiment, a nucleic acid fragment of the invention comprises the full length coding sequence of a mutant delta-12 or mutant delta-15 fatty acid desaturase.

In some embodiments, the sequence of a nucleic acid fragment may comprise more than one mutation or more than one type of mutation. Suitable types of mutations include, without limitation, insertions of nucleotides, deletions of nucleotides, or transitions and transversions in the wild-type coding sequence. Such mutations result in insertions of one or more amino acids, deletions of one or more amino acids, and non-conservative amino acid substitutions in the corresponding gene product.

Insertion or deletion of amino acids in a coding sequence may, for example, disrupt the conformation of essential alpha-helical or beta-pleated sheet regions of the resulting gene product. Amino acid insertions or deletions may also disrupt binding or catalytic sites important for gene product activity. It is known in the art that the insertion or deletion of a larger number of contiguous amino acids is more likely to render the gene product non-functional, compared to a smaller number of inserted or deleted amino acids.

Non-conservative amino acid substitutions may replace an amino acid of one class with an amino acid of a different class. Non-conservative substitutions may make a substantial change in the charge or hydrophobicity of the gene product. Non-conservative amino acid substitutions may also make a substantial change in the bulk of the residue side chain, e.g., substituting an alanyl residue for a isoleucyl residue.

Examples of non-conservative substitutions include. the substitution of a basic amino acid for a non-polar amino acid, or a polar amino acid for an acidic amino acid. Because there are only 20 amino acids encoded in a gene, substitutions that result in a non-functional gene product may be determined by routine experimentation, incorporating amino acids of a different class in the region of the gene product targeted for mutation.

Preferred mutations are in a region of the nucleic acid having an amino acid sequence motif that is conserved among delta-12 fatty acid desaturases or delta-15 fatty acid desaturases, such as a His-Xaa-Xaa-Xaa-His motif (Tables 1-3). An example of a suitable region has a conserved HECGH motif that is found, for example, in nucleotides corresponding to amino acids 105 to 109 of the *Arabidopsis* and *Brassica* delta-12 desaturase sequences, in nucleotides corresponding to amino acids 101 to 105 of the soybean delta-12 desaturase sequence and in nucleotides corresponding to amino acids 111 to 115 of the maize delta-12 desaturase sequence. See e.g., WO/11516; Okuley et al., Plant Cell 6:147-158 (1994). The one letter amino acid designations used herein are described in Alberts, B. et al., Molecular Biology of the Cell, 3rd edition, Garland Publishing, New York, 1994. Amino acids flanking this motif are also highly conserved among delta-12 and delta-15 desaturases and are also suitable candidates for mutations in fragments of the invention. The mutation recited in the claims is a Glu to Lys substitution in the HECGH motif of a delta-12 desaturase sequence. Preferably, the delta-12 desaturase gene is a Brassica microsomal delta-12 desaturase gene, either the D form or the F form. This mutation results in the sequence HECGH being changed to HKCGH as seen by comparing SEQ ID NO:2 (wild-type D form) to SEQ ID NO:4 (mutant D form).

A similar motif may be found at amino acids 101 to 105 of the Arabidopsis microsomal delta-15 fatty acid desaturase, as well as in the corresponding rape and soybean desaturases (Table 5). See, e.g., WO 93/11245; Arondel, V. et al., Science, 258:1153-1155 (1992); Yadav, N. et al., Plant Physiol., 103:467-476 (1993). Plastid delta-15 fatty acids have a similar motif (Table 5).

Among the types of mutations in an HECGH motif that render the resulting gene product non-functional are non-conservative substitutions. An illustrative example of a non-conservative substitution is substitution of a glycine residue for either the first or second histidine. Such a substitution replaces a polar residue (histidine) with a non-polar residue (glycine). Another type of mutation that renders the resulting gene product non-functional is an insertion mutation, e.g., insertion of a glycine between the cysteine and glutamic acid residues in the HECGH motif.

Other regions having suitable conserved amino acid motifs include the HRRHH motif shown in Table 2, the HRTHH motif shown in Table 6 and the HVAHH motif shown in Table 3. See, e.g., WO 94/11516; Hitz, W. et al., Plant Physiol., 105:635-641 (1994); Okuley, J., et al., supra; and Yadav, N. et al., supra.

Another region suitable for a mutation in a delta-12 desaturase sequence contains the motif KYLNNP at nucleotides corresponding to amino acids 171 to 175 of the *Brassica* desaturase sequence. An illustrative example of a mutation is this region is a Leu to His substitution, resulting in the amino acid sequence (Table 4) KYHNN (Compare wild-type SEQ ID NO:6 to mutant SEQ ID NO:8).

**TABLE 1 Alignment of Amino Acid Sequences from Microsomal Delta-12 Fatty Acid Desaturases**

| Species | Position | Amino Acid Sequence |
|---|---|---|
| *Arabidopsis thaliana* | 100-129 | IWVIAHECGH HAFSDYQWLD DTVGLIFHSF |
| *Glycine max* | 96-125 | VWVIAHECGH HAFSKYQWVD DVVGLTLHST |
| *Zea mays* | 106-135 | VWVIAHECGH HAFSDYSLLD DWGLVLHSS |
| *Ricinus communis*^{a} | 1- 29 | WVMAHDCGH HAFSDYQLLD DVVGLILHSC |
| *Brassica napus D* | 100-128 | VWVIAHECGH HAFSDYQWLD DTVGLIFHS |
| *Brassica napus F* | 100-128 | VWVIAHECGH HAFSDYQWLD DTVGLIFHS |

| | | |
|---|---|---|
| ^{a} from plasmid pRF2-1C | | |

**TABLE 2 Alignment of Amino Acid Sequences from Microsomal Delta-12 Fatty Acid Desaturases**

| Species | Position | Amino Acid Sequence |
|---|---|---|
| *Arabidopsis thaliana* | 130-158 | LLVPYFSWKY SHRRHHSNTG SLERDEVFV |
| *Glycine max* | 126-154 | LLVPYFSWKI SHRRHHSNTG SLDRDEVFV |
| *Zea mays* | 136-164 | LMVPYFSWKY SHRRHHSNTG SLERDEVFV |
| *Ricinus communis*^{a} | 30- 58 | LLVPYFSWKH SHRRHHSNTG SLERDEVFV |
| *Brassica napus D* | 130-158 | LLVPYFSWKY SHRSHHSNTG SLERDEVFV |
| *Brassica napus F* | 130-158 | LLVPYFSWKY SHRRHHSNTG SLERDEVFV |

| | | |
|---|---|---|
| ^{a} from plasmid pRF2-1C | | |

**TABLE 3 Alignment of Amino Acid Sequences from Microsomal Delta-12 Fatty Acid Desaturases**

| Species | Position | Amino Acid Sequence |
|---|---|---|
| *Arabidopsis thaliana* | 298-333 | DRDYGILNKV FHNITDTHVA HHLFSTMPHY NAMEAT |
| *Glycine max* | 294-329 | DRDYGILNKV FHHITDTHVA HHLFSTMPHY HAMEAT |
| *Zea mays* | 305-340 | DRDYGILNRV FHNITDTHVA HHLFSTMPHY HAMEAT |
| *Ricinus communis*^{a} | 198-224 | DRDYGILNKV FHNITDTQVA HHLF TMP |
| *Brassica napus D* | 299-334 | DRDYGILNKV FHNITDTHVA HHPFSTMPHY HAMEAT |
| *Brassica napus F* | 299-334 | DRDYGILNKV FHNITDTHVA HHLPSTMPHY HAMEAT |

| | | |
|---|---|---|
| ^{a}from plasmid pRF2-1C | | |

**TABLE 4 Alignment of Conserved Amino Acids from Microsomal Delta-12 Fatty Acid Desaturases**

| Species | Position | Amino Acid Sequence |
|---|---|---|
| *Arabidopsis thaliana* | 165-180 | IKWYGKYLNN PLGRIM |
| *Glycine max* | 161-176 | VAWFSLYLNN PLGRAV |
| *Zea mays* | 172-187 | PWYTPYVYNN PVGRVV |
| *Ricinus communis*^{a} | 65- 80 | IRWYSKYLNN PPGRIM |
| *Brassica napus* D | 165-180 | IKWYGKYLNN PLGRTV |
| *Brassica napus* F | 165-180 | IKWYGKYLNN PLGRTV |

| | | |
|---|---|---|
| ^{a} from plasmid pRF2-1C | | |

**TABLE 5 Alignment of Conserved Amino Acids from Plastid and Microsomal Delta-15 Fatty Acid Desaturases**

| Species | Position | Amino Acid Sequence |
|---|---|---|
| *Arabidopsis thaliana*^{a} | 156-177 | WALFVLGHD CGHGSFSNDP KLN |
| *Brassica napus*^{a} | 114-135 | WALFVLGHD CGHGSFSNDP RLN |
| *Glycine max*^{a} | 164-185 | WALFVLGHD CGHGSFSNNS KLN |
| *Arabidopsis thaliana* | 94-115 | WAIFVLGHD CGHGSFSDIP LLN |
| *Brassica napus* | 87-109 | WALFVLGHD CGHGSFSNDP RLN |
| *Glycine max* | 93-114 | WALFVLGHD CGHGSFSDSP PLN |

| | | |
|---|---|---|
| ^{a} Plastid sequences | | |

**TABLE 6 Alignment of Conserved Amino Acids from Plastid and Microsomal Delta-15 Fatty Acid Desaturases**

| Species | Position | Amino Acid Sequence |
|---|---|---|
| *A. thaliana*^{a} | 188-216 | ILVPYHGWRI SHRTHHQNHG HVENDESWH |
| *B. napus*^{a} | 146-174 | ILVPYHGWRI SHRTHHQNHG HVENDESWH |
| *Glycine max*^{a} | 196-224 | ILVPYHGWRI SHRTHHQHHG HAENDESWH |
| *A. thaliana* | 126-154 | ILVPYHGWRI SHRTHHQNHG HVENDESWV |
| *Brassica napus* | 117-145 | ILVPYHGWRI SHRTHHQNHG HVENDESWV |
| *Glycine max* | 125-153 | ILVPYHGWRI SHRTHHQNHG HIEKDESWV |

| | | |
|---|---|---|
| ^{a}Plastid sequences | | |

The conservation of amino acid motifs and their relative positions indicates that regions of a delta-12 or delta-15 fatty acid desaturase that can be mutated in one species to generate a non-functional desaturase can be mutated in the corresponding region from other species to generate a non-functional 12-DES or 15-DES gene product in that species.

A nucleic acid fragment containing a mutant sequence can be generated by techniques known to the skilled artisan. Such techniques include, without limitation, site-directed mutagenesis of wild-type sequences and direct synthesis using automated DNA synthesizers.

A nucleic acid fragment containing a mutant sequence can also be generated by mutagenesis of plant seeds or regenerable plant tissue by, e.g., ethyl methane sulfonate, X-rays or other mutagens. With mutagenesis, mutant plants having the desired fatty acid phenotype in seeds are identified by known techniques and a nucleic acid fragment containing the desired mutation is isolated from genomic DNA or RNA of the mutant line. The site of the specific mutation is then determined by sequencing the coding region of the 12-DES or 15-DES gene. Alternatively, labeled nucleic acid probes that are specific for desired mutational events can be used to rapidly screen a mutagenized population.

Seeds of Westar, a Canadian (*Brassica napus*) spring canola variety, were subjected to chemical mutagenesis. Mutagenized seeds were planted in the greenhouse and the plants were self-pollinated. The progeny plants were individually analyzed for fatty acid composition, and regrown either in the greenhouse or in the field. After four successive generations of self-pollinations, followed by chemical analysis of the seed oil at each cycle, several lines were shown to carry stably inherited mutations in specific fatty acid components, including reduced palmitic acid (C_{16:0}), increased palmitic acid, reduced stearic acid (C_{18:0}), increased oleic acid (C_{18:1}), reduced linoleic acid (C_{18.2}) and reduced linolenic acid (C_{18.3}), in the seed oil.

The general experimental scheme for developing lines with stable fatty acid mutations is shown in Scheme I hereinafter.

### STABLE FATTY ACID MUTANTS

Westar seeds (M₀) were mutagenized with ethylmethanesulfonate (EMS). Westar is a registered Canadian spring variety with canola quality. The fatty acid composition of field-grown Westar, 3.9% C_{16:0}, 1.9% C_{18:0}, 67.5% C_{18:1}, 17.6% C_{18:2}, 7.4% C_{18:3}, <2% C20:1 + C_{22:1}, has remained stable under commercial production, with <± 10% deviation, since 1982. The disclosed method may be applied to all oilseed *Brassica* species, and to both Spring and Winter maturing types within each species. Physical mutagens, including but not limited to X-rays, UV rays, and other physical treatments which cause chromosome damage, and other chemical mutagens, including but not limited to ethidium bromide, nitrosoguanidine, diepoxybutane etc. may also be used to induce mutations. The mutagenesis treatment may also be applied to other stages of plant development, including but not limited to cell cultures, embryos, microspores and shoot apices. The M₁ seeds were planted in the greenhouse and M₁ plants were individually self-pollinated.

M₂ seed was harvested from the greenhouse and planted in the field in a plant-to-row design. Each plot contained six rows, and five M₂ lines were planted in each plot. Every other plot contained a row of non-mutagenized Westar as a control. Based on gas chromatographic analysis of M₂ seed, those lines which had altered fatty acid composition were self-pollinated and individually harvested.

M₃ seeds were evaluated for mutations on the basis of a Z-distribution. An extremely stringent 1 in 10,000 rejection rate was employed to establish statistical thresholds to distinguish mutation events from existing variation. Mean and standard deviation values were determined from the non-mutagenized Westar control population in the field. The upper and lower statistical thresholds for each fatty acid were determined from the mean value of the population ± the standard deviation, multiplied by the Z-distribution. Based on a population size of 10,000, the confidence interval is 99.99%.

Seeds (M₃) from those M₂ lines which exceeded either the upper or lower statistical thresholds were replanted in the greenhouse and self-pollinated. This planting also included Westar controls. The M₄ seed was re-analyzed using new statistical thresholds established with a new control population. Those M, lines which exceeded the new statistical thresholds for selected fatty acid compositions were advanced to the nursery. Following self-pollination, M₅ seed from the field were re-analyzed once again for fatty acid composition. Those lines which remained stable for the selected fatty acids were considered stable mutations.

"Stable mutations" as used herein are defined as M₅ or more advanced lines which maintain a selected altered fatty acid profile for a minimum of three generations, including a minimum of two generations under field conditions, and exceeding established statistical thresholds for a minimum of two generations, as determined by gas chromatographic analysis of a minimum of 10 randomly selected seeds bulked together. Alternatively, stability may be measured in the same way by comparing to subsequent generations. In subsequent generations, stability is defined as having similar fatty acid profiles in the seed as that of the prior or subsequent generation when grown under substantially similar conditions.

The amount of variability for fatty acid content in a seed population is quite significant when single seeds are analyzed. Randomly selected single seeds and a ten seed bulk sample of a commercial variety were compared. Significant variation among the single seeds was detected (Table A). The half-seed technique (Downey, R.K. and B.L. Harvey, Can. J. Plant Sci., 43:271 [1963]) in which one cotyledon of the germinating seed is analyzed for fatty acid composition and the remaining embryo grown into a plant has been very useful to plant . breeding work to select individuals in a population for further generation analysis. The large variation seen in the single seed analysis (Table A) is reflected in the half-seed technique.

**TABLE A Single Seed Analvsis for Fatty Acid Composition¹**

| | | | | | | | | | | |
|---|---|---|---|---|---|---|---|---|---|---|
| SAMPLE | 16:0 | 16:1 | 18:0 | 18:1 | 18:2 | 18:3 | 20:0 | 20:1 | 22:0 | 22:1 |
| Bulk | 3.2 | 0.4 | 1.8 | 20.7 | 13.7 | 9.8 | 0.8 | 11.2 | 0.4 | 32.2 |
| 1 | 2.8 | 0.2 | 1.1 | 14.6 | 14.6 | 11.1 | 0.8 | 9.8 | 0.7 | 38.2 |
| 2 | 3.3 | 0.2 | 1.3 | 13.1 | 14.4 | 11.7 | 0.9 | 10.5 | 0.7 | 37.0 |
| 3 | 3.0 | -- | 1.2 | 12.7 | 15.3 | 10.6 | 0.8 | 7.3 | 0.7 | 43.2 |
| 4 | 2.8 | 0.2 | 1.1 | 16.7 | 13.2 | 9 1 | 0.8 | 11.2 | 0.4 | 38.9 |
| 5 | 3.0 | -- | 1.8 | 15.2 | 13.3 | 8.4 | 1.3 | 8.7 | 0.9 | 42.3 |
| 6 | 3.1 | -- | 1.3 | 14.4 | 14.6 | 10.3 | 1.0 | 10.9 | 0.8 | 39.3 |
| 7 | 2.6 | -- | 1.2 | 15.7 | 13.8 | 9.9 | 0.9 | 12.2 | 0.5 | 37.0 |
| 8 | 3.1 | -- | 1.1 | 16.2 | 13.4 | 10.6 | 0.6 | 9.2 | 0.8 | 41.4 |
| 9 | 2.7 | 0.1 | 1.0 | 13.5 | 11.2 | 11.3 | 0.8 | 6.2 | 0.7 | 46.9 |
| 10 | 3.4 | 0.2 | 1.4 | 13.9 | 17.5 | 10.8 | 1.1 | 10.0 | 0.9 | 36.2 |
| 11 | 2.8 | 0.2 | 1.2 | 12.7 | 12.9 | 10.3 | 1.0 | 7.9 | 0.9 | 43.3 |
| 12 | 2.3 | 0.1 | 1.6 | 20.7 | 14.8 | 6.5 | 1.1 | 12.5 | 0.8 | 34.5 |
| 13 | 2.6 | 0.2 | 1.3 | 21.0 | 11.4 | 7.6 | 1.0 | 11.6 | 0.6 | 36.7 |
| 14 | 2.6 | 0.1 | 1.2 | 14.7 | 13.2 | 9.4 | 0.9 | 10.1 | 0.8 | 40.8 |
| 15 | 2.9 | 0.2 | 1.4 | 16.6 | 15.1 | 11.2 | 0.7 | 9.1 | 0.3 | 36.1 |
| 16 | 3.0 | 0.2 | 1.1 | 12.4 | 13.7 | 10.4 | 0.9 | 8.7 | 0.8 | 42.7 |
| 17 | 2.9 | 0.1 | 1.1 | 21.1 | 12.3 | 7.1 | 0.8 | 12.4 | 0.5 | 36.8 |
| 18 | 3.1 | 0.1 | 1.2 | 13.7 | 13.1 | 10.4 | 1.0 | 8.8 | 0.7 | 41.6 |
| 19 | 2.7 | 0.1 | 1.0 | 11.1 | 13.4 | 11.7 | 0.8 | 7.9 | 0.8 | 43.5 |
| 20 | 2.3 | 0.2 | 0.2 | 18.2 | 13.9 | 8.2 | 0.9 | 10.3 | 0.8 | 38.2 |
| Average | 2.8 | 0.2 | 1.2 | 15.4 | 13.8 | 9.8 | 0.9 | 9.8 | 0.7 | 39.7 |
| Minimum | 2.3 | 0.1 | 0.2 | 11.1 | 11.2 | 6.5 | 0.6 | 6.2 | 0.3 | 34.5 |
| Maximum | 3.4 | 0.2 | 1.8 | 21.1 | 17.5 | 11.7 | 1.3 | 12.5 | 0.9 | 46.9 |
| Range | 1.1 | 0.1 | 1.6 | 9.9 | 6.3 | 5.3 | 0.7 | 6.4 | 0.6 | 12.4 |

| | | | | | | | | | | |
|---|---|---|---|---|---|---|---|---|---|---|
| Values expressed as percent of total oil | | | | | | | | | | |

Plant breeders using the half-seed technique have found it unreliable in selecting stable genetically controlled fatty acid mutations (Stefanson, B.R., In; High and Low Erucic Acid Rapeseed Oils, Ed. N.T. Kenthies, Academic Press, Inc., Canada (1983) pp. 145-159). Although valuable in selecting individuals from a population, the selected traits are not always transmitted to subsequent generations (Rakow, G. and McGregor, D.I., J. Amer. Oil Chem. Soc. (1973) 50:400-403. To determine the genetic stability of the selected plants several self-pollinated generations are required (Robelen, G. In: Biotechnology for the Oils and Fats Industry, Ed. C. Ratledge, P. Dawson and J. Rattray, American Oil Chemists Society (1984) pp. 97-105) with chemical analysis of a bulk seed sample.

Mutation breeding has traditionally produced plants carrying, in addition to the trait of interest, multiple, deleterious traits, e.g., reduced plant vigor and reduced fertility. Such traits may indirectly affect fatty acid composition, producing an unstable mutation; and/or reduce yield, thereby reducing the commercial utility of the invention. To eliminate the occurrence of deleterious mutations and reduce the load of mutations carried by the plant a low mutagen dose was used in the seed treatments to create an LD30 population. This allowed for the rapid selection of single gene mutations for fatty acid traits in agronomic backgrounds which produce acceptable yields.

Other than changes in the fatty acid composition of the seed oil, the mutant lines described here have normal plant phenotype when grown under field conditions, and are commercially useful. "Commercial utility" is defined as having a yield, as measured by total pounds of seed or oil produced per acre, within 15% of the average yield of the starting (M₀) canola variety grown in the same region. To be commercially useful, plant vigor and high fertility are such that the crop can be produced in this yield by farmers using conventional farming equipment, and the oil with altered fatty acid composition can be extracted using conventional crushing and extraction equipment.

The seeds of several different fatty acid lines have been deposited with the American Type Culture Collection and have the following accession numbers.

| Line | Accession No. | Deposit Date |
|---|---|---|
| A129.5 | 40811 | May 25, 1990 |
| A133.1 | 40812 | May 25, 1990 |
| A144.1 | 40813 | May 25, 1990 |
| A200.7 | 40816 | May 31, 1990 |
| M3032.1 | 75021 | June 7, 1991 |
| M3094.4 | 75023 | June 7, 1991 |
| M3052.6 | 75024 | June 7, 1991 |
| M3007.4 | 75022 | June 7, 1991 |
| M3062.8 | 75025 | June 7, 1991 |
| M3028.10 | 75026 | June 7, 1991 |
| IMC130 | 75446 | April 16, 1993 |

In some plant species or varieties more than one form of endogenous microsomal delta-12 desaturase may be found. In amphidiploids, each form may be derived from one of the parent genomes making up the species under consideration. Plants with mutations in both forms have a fatty acid profile that differs from plants with a mutation in only one form. An example of such a plant is *Brassica napus* line *Q508*, a doubly-mutagenized line containing a mutant D-form of delta-12 desaturase (SEQ ID NO:1) and a mutant F-form of delta-12 desaturase (SEQ ID NO:5).

Preferred host or recipient organisms for introduction of a nucleic acid fragment of the invention are the oil-producing species, such as soybean *(Glycine max*), rapeseed (e.g., *Brassica napus, B. rapa* and *B. juncea*)*,* sunflower (*Helianthus annus*), castor bean (*Ricinus communis*)*,* corn (*Zea mays*)*,* and safflower (*Carthamus tinctorius*).

Plants according to the invention preferably contain an altered fatty acid profile. For example, oil obtained from seeds of such plants may have from about 69 to about 90% oleic acid, based on the total fatty acid composition of the seed. Such oil preferably has from about 74 to about 90% oleic acid, more preferably from about 80 to about 90% oleic acid. In some embodiments, oil obtained from seeds produced by plants of the invention may have from about 2.0% to about 5.0% saturated fatty acids, based on total fatty acid composition of the seeds. In some embodiments, oil obtained from seeds of the invention may have from about 1.0% to about 14.0% linoleic acid, or from about 0.5% to about 10.0% α-linolenic acid.

In one embodiment of the claimed invention, a plant contains both a 12-DES mutation and a 15-DES mutation. Such plants can have a fatty acid composition comprising very high oleic acid and very low alpha-linolenic acid levels. Mutations in 12-DES and 15-DES may be combined in a plant by making a genetic cross between 12-DES and 15-DES single mutant lines. A plant having a mutation in delta-12 fatty acid desaturase is crossed or mated with a second plant having a mutation in delta-15 fatty acid desaturase. Seeds produced from the cross are planted and the resulting plants are selfed in order to obtain progeny seeds. These progeny seeds are then screened in order to identify those seeds carrying both mutant genes.

Alternatively, a line possessing either a 12-DES or a 15-DES mutation can be subjected to mutagenesis to generate a plant or plant line having mutations in both 12-DES and 15-DES. For example, the IMC 129 line has a mutation in the coding region (Glu₁₀₆ to Lys₁₀₆) of the D form of the microsomal delta-12 desaturase structural gene. Cells (e.g., seeds) of this line can be mutagenized to induce a mutation in a 15-DES gene, resulting in a plant or plant line carrying a mutation in a delta-12 fatty acid desaturase gene and a mutation in a delta-15 fatty acid desaturase gene.

Progeny includes descendants of a particular plant or plant line, e.g., seeds developed on an instant plant. Progeny of an instant plant include seeds formed on F₁, F₂, F₃, and subsequent generation plants, or seeds formed on BC₁, BC₂, BC₃ and subsequent generation plants.

Those seeds having an altered fatty acid composition may be identified by techniques known to the skilled artisan, e.g., gas-liquid chromatography (GLC) analysis of a bulked seed sample or of a single half-seed. Half-seed analysis is well known in the art to be useful because the viability of the embryo is maintained and thus those seeds having a desired fatty acid profile may be planted to from the next generation. However, half-seed analysis is also known to be an inaccurate representation of genotype of the seed being analyzed. Bulk seed analysis typically yields a more accurate representation of the fatty acid profile of a given genotype.

The nucleic acid fragments of the invention can be used as markers in plant genetic mapping and plant breeding programs. Such markers may include restriction fragment length polymorphism (RFLP), random amplification polymorphism detection (RAPD), polymerase chain reaction (PCR) or self-sustained sequence replication (3SR) markers, for example. Marker-assisted breeding techniques may be used to identify and follow a desired fatty acid composition during the breeding process. Marker-assisted breeding techniques may be used in addition to, or as an alternative to, other sorts of identification techniques. An example of marker-assisted breeding is the use of PCR primers that specifically amplify a sequence containing a desired mutation in 12-DES or 15-DES.

Methods according to the invention are useful in that the resulting plants and plant lines have desirable seed fatty acid compositions as well as superior agronomic properties compared to known lines having altered seed fatty acid composition. Superior agronomic characteristics include, for example, increased seed germination percentage, increased seedling vigor, increased resistance to seedling fungal diseases (damping off, root rot and the like), increased yield, and improved standability.

While the invention is susceptible to various modifications and alternative forms, certain specific embodiments thereof are described in the general methods and examples set forth below. For example the invention may be applied to all *Brassica* species, including B. *rapa, B. juncea*, and *B. hirta*, to produce substantially similar results. It should be understood, however, that these examples are not intended to limit the invention to the particular forms disclosed but, instead the invention is to cover all modifications, equivalents and alternatives falling within the scope of the invention. This includes the use of somaclonal variation; physical or chemical mutagenesis of plant parts; anther, microspore or ovary culture followed by chromosome doubling; or self- or cross-pollination to transmit the fatty acid trait, alone or in combination with other traits, to develop new *Brassica* lines.

### EXAMPLE 1

### Selection of Low FDA Saturates

Prior to mutagenesis, 30,000 seeds of *B*. *napus* cv. Westar seeds were preimbibed in 300-seed lots for two hours on wet filter paper to soften the seed coat. The preimbibed seeds were placed in 80 mM ethylmethanesulfonate (EMS) for four hours. Following mutagenesis, the seeds were rinsed three times in distilled water. The seeds were sown in 48-well flats containing Pro-Mix. Sixty-eight percent of the mutagenized seed germinated. The plants were maintained at 25°C/15°C, 14/10 hr day/night conditions in the greenhouse. At flowering, each plant was individually self-pollinated.

M₂ seed from individual plants were individually catalogued and stored, approximately 15,000 M₂ lines was planted in a summer nursery in Carman, Manitoba. The seed from each selfed plant were planted in 3-meter rows with 6-inch row spacing. Westar was planted as the check variety. Selected lines in the field were selfed by bagging the main raceme of each plant. At maturity, the selfed plants were individually harvested and seeds were catalogued and stored to ensure that the source of the seed was known.

Self-pollinated M₃ seed and Westar controls were analyzed in 10-seed bulk samples for fatty acid composition via gas chromatography. Statistical thresholds for each fatty acid component were established using a Z-distribution with a stringency level of 1 in 10,000. The selected M₃ seeds were planted in the greenhouse along with Westar controls. The seed was sown in 4-inch pots containing Pro-Mix soil and the plants were maintained at 25°C/15°C, 14/10 hr day/night cycle in the greenhouse. At flowering, the terminal raceme was self-pollinated by bagging. At maturity, selfed M₄ seed was individually harvested from each plant, labelled, and stored to ensure that the source of the seed was known.

The M₄ seed was analyzed in 10-seed bulk samples. Statistical thresholds for each fatty acid component were established from 259 control samples using a Z-distribution of 1 in 800. Selected M, lines were planted in a field trial in Carman, Manitoba in 3-meter rows with 6-inch spacing. Ten M₄ plants in each row were bagged for self-pollination. At maturity, the selfed plants were individually harvested and the open pollinated plants in the row were bulk harvested. The M₅ seed from single plant selections was analyzed in 10-seed bulk samples and the bulk row harvest in 50-seed bulk samples.

Selected M₅ lines were planted in the greenhouse along with Westar controls. The seed was grown as previously described. At flowering the terminal raceme was self-pollinated by bagging. At maturity, selfed M₆ seed was individually harvested from each plant and analyzed in 10-seed bulk samples for fatty acid composition.

Selected M₆ lines were entered into field trials in Eastern Idaho. The four trial locations were selected for the wide variability in growing conditions. The locations included Burley, Tetonia, Lamont and Shelley (Table I). The lines were planted in four 3-meter rows with an 8-inch spacing, each plot was replicated four times. The planting design was determined using a Randomized Complete Block Designed. The commercial cultivar Westar was used as a check cultivar. At maturity the plots were harvested to determine yield. Yield of the entries in the trial was determined by taking the statistical average of the four replications. The Least Significant Difference Test was used to rank the entries in the randomized complete block design.

**TABLE I Trial Locations for Selected Fatty Acid Mutants**

| LOCATION | SITE CHARACTERIZATIONS |
|---|---|
| BURLEY | Irrigated. Long season. High temperatures during flowering. |
| TETONIA | Dryland. Short season. Cool temperatures. |
| LAMONT | Dryland. Short season. Cool temperatures. |
| SHELLEY | Irrigated. Medium season. High temperatures during flowering. |

To determine the fatty acid profile of entries, plants in each plot were bagged for self-pollination. The M₇ seed from single plants was analyzed for fatty acids in ten-seed bulk samples.

To determine the genetic relationships of the selected fatty acid mutants crosses were made. Flowers of M₆ or later generation mutations were used in crossing. F₁ seed was harvested and analyzed for fatty acid composition to determine the mode of gene action. The F₁ progeny were planted in the greenhouse. The resulting plants were self-pollinated, the F₂ seed harvested and analyzed for fatty acid composition for allelism studies. The F₂ seed and parent line seed was planted in the greenhouse, individual plants were self-pollinated. The F₃ seed of individual plants was tested for fatty acid composition using 10-seed bulk samples as described previously.

In the analysis of some genetic relationships dihaploid populations were made from the microspores of the F₁ hybrids. Self-pollinated seed from dihaploid plants were analyzed for fatty acid analysis using methods described previously.

For chemical analysis, 10-seed bulk samples were hand ground with a glass rod in a 15-mL polypropylene tube and extracted in 1.2 mL 0.25 N KOH in 1:1 ether/methanol. The sample was vortexed for 30 sec. and heated for 60 sec. in a 60°C water bath. Four mL of saturated NaCl and 2.4 mL of iso-octane were added, and the mixture was vortexed again. After phase separation, 600 µL of the upper organic phase were pipetted into individual vials and stored under nitrogen at -5°C. One µL samples were injected into a Supelco SP-2330 fused silica capillary column (0.25 mm ID, 30 M length, 0.20 µm df).

The gas chromatograph was set at 180°C for 5.5 minutes, then programmed for a 2°C/minute increase to 212°C, and held at this temperature for 1.5 minutes. Total run time was 23 minutes. Chromatography settings were: Column head pressure - 15 psi, Column flow (He) - 0.7 mL/min., Auxiliary and Column flow - 33 mL/min., Hydrogen flow - 33 mL/min., Air flow - 400 mL/min., Injector temperature - 250°C, Detector temperature - 300°C, Split vent - 1/15.

Table II describes the upper and lower statistical thresholds for each fatty acid of interest.

**TABLE II Statistical Thresholds for Specific Fatty Acids Derived from Control Westar Plantings**

| | | | Percent Fatty Acids | | | |
|---|---|---|---|---|---|---|
| Genotype | C_{16:0} | C_{18.0} | C_{18.1} | C_{18:2} | C_{18:3} | Sats* |
| M₃ Generation(1 in 10,000 rejection rate) | | | | | | |
| Lower | 3.3 | 1.4 | -- | 13.2 | 5.3 | 6.0 |
| Upper | 4.3 | 2.5 | 71.0 | 21.6 | 9.9 | 8.3 |

| M₄ Generation(1 in 800 rejection rate) | | | | | | |
|---|---|---|---|---|---|---|
| Lower | 3.6 | 0.8 | -- | 12.2 | 3.2 | 5.3 |
| Upper | 6.3 | 3.1 | 76.0 | 32.4 | 9.9 | 11.2 |

| M₅ Generation (1 in 755 rejection rate) | | | | | | |
|---|---|---|---|---|---|---|
| Lower | 2.7 | 0.9 | -- | 9.6 | 2.6 | 4.5 |
| Upper | 5.7 | 2.7 | 80.3 | 26.7 | 9.6 | 10.0 |

| | | | | | | |
|---|---|---|---|---|---|---|
| *Sats=Total Saturate Content | | | | | | |

At the M₃ generation, twelve lines exceeded the lower statistical threshold for palmitic acid (≤3.3%). Line W13097.4 had 3.1% palmitic acid and an FDA saturate content of 4.5%. After a cycle in the greenhouse, M₄ seed from line W13097.4 (designated line A144) was analyzed. Line W13097.4.1(A144.1) had 3.1% C_{16:0}, exceeding the lower statistical threshold of 3.6%. The FDA saturate content for A144.1 was 4.5%. The fatty acid compositions for the M₃, M₄ and M₅ generations of this family are summarized in Table III.

**TABLE III Fatty Acid Composition of a Low Palmitic Acid/Low FDA Saturate Canola Line Produced by Seed Mutaqenesis**

| | | | | Percent Fatty Acids | | | | |
|---|---|---|---|---|---|---|---|---|
| Genotype^{a} | C_{16:0} | C_{18:0} | C_{18:1} | C_{18:2} | C_{18:3} | Sats^{b} | Tot Sat^{c} | |
| Westar | 3.9 | 1.9 | 67.5 | 17.6 | 7.4 | 5.9 | 7.0 | |
| | | | | | | | | |
| W13097.4 | | | | | | | | |
| (M₃) | | | | 18.6 | 9.5 | 4.5 | 5.6 | |
| W13097.4 | 3.1 | 1.4 | 63.9 | | | | | |
| (M₄) | 3.1 | 1.4 | 66.2 | 19.9 | 6.0 | 4.5 | 5.5 | |
| A144.1.9 | | | | | | | | |
| (M₅) | 2.9 | 1.4 | 64.3 | 20.7 | 7.3 | 4.4 | 5.3 | |

| | | | | | | | | |
|---|---|---|---|---|---|---|---|---|
| ^{a}Letter and numbers up to second decimal point indicate the plant line. Number after second decimal point indicates an individual plant. | | | | | | | | |
| ^{b}Sat=FDA Saturates | | | | | | | | |
| ^{c}Tot Sat=Total Saturate Content | | | | | | | | |

The M₅ seed of ten self-pollinated A144.1 (ATCC 40813) plants averaged 3.1% palmitic acid and 4.7% FDA saturates. One selfed plant (A144.1.9) contained 2.9% palmitic acid and FDA saturates of 4.4%. Bulk seed analysis from open-pollinated (A144.1) plants at the M₅ generation averaged 3.1% palmitic acid and 4.7% FDA saturates. The fatty acid composition of the bulked and individual A144.1 lines are summarized in Table IV.

**TABLE IV Fatty Acid Composition of A144 Low Palmitic Acid/Low FDA Saturate Line**

| | | | | Percent Fatty Acids | | | | |
|---|---|---|---|---|---|---|---|---|
| Genotype^{a} | C_{16:0} | C_{18:0} | C_{18:1} | C_{18:2} | C_{18.3} | Sats^{b} | Tot Sat^{c} | |
| Individually Self-Pollinated Plants | | | | | | | | |
| A144.1.1 | 3.2 | 1.6 | 64.4 | 20.5 | 7.0 | 4.8 | 5.9 | |
| A144.1.2 | 3.0 | 1.5 | 67.4 | 18.6 | 6.3 | 4.5 | 5.7 | |
| A144.1.3 | 3.6 | 1.8 | 61.4 | 22.4 | 7.5 | 5.2 | 6.6 | |
| A144.1.4 | 3.2 | 1.5 | 64.6 | 20.9 | 6.7 | 4.7 | 5.8 | |
| A144.1.5 | 3.3 | 1.7 | 60.0 | 23.9 | 7.9 | 5.0 | 6.1 | |
| A144.1.6 | 3.1 | 1.4 | 67.3 | 17.8 | 6.5 | 4.6 | 5.2 | |
| A144.1.7 | 3.1 | 1.6 | 67.7 | 17.4 | 6.5 | 4.8 | 5.4 | |
| A144.1.8 | 3.1 | 1.8 | 66.9 | 18.7 | 6.1 | 4.9 | 5.4 | |
| A144.1.9 | 2.9 | 1.4 | 64.3 | 20.7 | 7.3 | 4.4 | 5.3 | |
| A144.1.10 | 3.1 | 1.5 | 62.5 | 20.4 | 7.7 | 4.6 | 5.6 | |

| Average of Individually Self-Pollinated Plants | | | | | | | | |
|---|---|---|---|---|---|---|---|---|
| A144.1.1-10 | 3.1 | 1.6 | 64.8 | 20.1 | 6.9 | 4.7 | 5.7 | |

| Bulk Analysis of Open-Pollinated Plants | | | | | | | | |
|---|---|---|---|---|---|---|---|---|
| A144.1B | 3.1 | 1.6 | 64.8 | 19.4 | 7.8 | 4.7 | 5.7 | |

| | | | | | | | | |
|---|---|---|---|---|---|---|---|---|
| ^{a}Letter and numbers up to second decimal point indicate the plant line. Number after second decimal point indicates an individual plant. | | | | | | | | |
| ^{b}Sat=FDA Saturates | | | | | | | | |
| ^{c}tot Sat=Total Saturate Content | | | | | | | | |

These reduced levels have remained stable to the M₇ generations in both greenhouse and field conditions. These reduced levels have remained stable to the M₇ generation in multiple location field trails. Over all locations, the self-pollinated plants (A144) averaged 2.9% palmitic acid and FDA saturates of 4.6%. The fatty acid composition of the A144 lines for each Idaho location are summarized in Table V. In the multiple location replicated trial the yield of A144 was not significantly different in yield from the parent cultivar Westar. By means of seed mutagenesis, the level of saturated fatty acids of canola *(B. napus*) was reduced from 5.9% to 4.6%. The palmitic acid content was reduced from 3.9% to 2.9%.

**TABLE V Fatty Acid Composition of a Mutant Low Palmitic Acid/Low FDA Saturate Canola Line at Different Field Locations in Idaho**

| | | | | Percent Fatty Acids | | | | |
|---|---|---|---|---|---|---|---|---|
| Trial Location | C_{16:0} | C_{18:0} | C_{18:1} | C_{18.2} | C_{18:3} Sats | | Tot Sats | |
| Burley | 2.9 | 1.3 | 62.3 | 20.6 | 10.3 | 4.2 | 5.0 | |
| Tetonia | 2.9 | 1.7 | 59.7 | 21.0 | 11.2 | 4.6 | 5.7 | |
| Lamont | 3.1 | 1.8 | 63.2 | 19.5 | 9.0 | 4.9 | 5.9 | |
| Shelley | 2.8 | 1.9 | 64.5 | 18.8 | 8.8 | 4.7 | 5.9 | |

To determine the genetic relationship of the palmitic acid mutation in A144 (C_{16:0} - 3.0%, C_{18:0} - 1.5%, C_{18:1} - 67.4%, C_{18:2} - 18.6%, C_{18:3} - 6.3%) to other fatty acid mutations it was crossed to A129 a mutant high oleic acid (C_{16:0} - 3.8%, C_{18:0} - 2.3%, C_{18.1} - 75.6%, C_{18:2} - 9.5%, C_{18.3} - 4.9%). Over 570 dihaploid progeny produced from the F₁ hybrid were harvested and analyzed for fatty acid composition. The results of the progeny analysis are summarized in Table VB. Independent segregation of the palmitic traits was observed which demonstrates that the genetic control of palmitic acid in A144 is different from the high oleic acid mutation in A129.

**TABLE VB Genetic Studies of Dihaploid Progeny of A144 X A129**

| | | Frequency | |
|---|---|---|---|
| Genotype | C_{16:0} Content(%) | Observed | Expected |
| p-p-p2-p2- | 3.0% | 162 | 143 |
| p+p+p2-p2- | 3.4% | 236 | 286 |
| p+p+p2+p2+ | 3.8% | 175 | 143 |

### EXAMPLE 2

An additional low FDA saturate line, designated A149.3 (ATCC 40814), was also produced by the method of Example 1. A 50-seed bulk analysis of this line showed the following fatty acid composition: C_{16:0} - 3.6%, C_{18:0} - 1.4%, C_{18:1} - 65.5%, C_{18.2} - 18.3%, C_{18:3} - 8.2%, FDA Sats - 5.0%, Total Sats - 5.9%. This line has also stably maintained its mutant fatty acid composition to the M₅ generation. In a multiple location replicated trial the yield of A149 was not significantly different in yield from the parent cultivar Westar.

### EXAMPLE 3

An additional low palmitic acid and low FDA saturate line, designated M3094.4 (ATCC 75023), was also produced by the method of Example 1. A 10-seed bulk analysis of this line showed the following fatty acid composition: C_{16:0} - 2.7%, C_{18:0} - 1.6%, C_{18.1} - 66.6%, C_{18:2} - 20.0%, C_{18:3} - 6.1%, C_{20:1} - 1.4%, C_{22.1} - 0.0%, FDA Saturate - 4.3%, Total Saturates - 5.2%. This line has stably maintained its mutant fatty acid composition to the M₅ generation. In a single replicated trial the yield of M3094 was not significantly different in yield from the parent cultivar.

M3094.4 was crossed to A144, a low palmitic acid mutation (Example 1) for allelism studies. Fatty acid composition of the F₂ seed showed the two lines to be allelic. The mutational events in A144 and M3094, although different in origin, are in the same gene.

### EXAMPLE 4

In the studies of Example 1, at the M₃ generation, 470 lines exceed the upper statistical threshold for palmitic acid (≥4.3%). One M₃ line, W14538.6, contained 9.2% palmitic acid. Selfed progenies of this line, since designated M3007.4 (ATCC 75022), continued to exceed to the upper statistical threshold for high palmitic acid at both the M₄ and M₅ generations with palmitic acid levels of 11.7% and 9.1%, respectively. The fatty acid composition of this high palmitic acid mutant, which was stable to the M₇ generation under both field and greenhouse conditions, is summarized in Table VI.

**TABLE VI Fatty Acid Composition of a High Palmitic Acid Canola Line Produced by Seed Mutagenesis**

| | | | Percent Fatty Acids | | | |
|---|---|---|---|---|---|---|
| Genotype | C_{16.0} | C_{18·0} | C_{18:1} | C_{18:2} | C_{18:3} | Sats* |
| Westar | 3.9 | 1.9 | 67.5 | 17.6 | 7.4 | 7.0 |
| W114538.6 | 8.6 | 1.6 | 56.4 | 20.3 | 9.5 | 10.2 |
| (M₃) | | | | | | |
| M3007.2 | 11.7 | 2.1 | 57.2 | 18.2 | 5.1 | 13.9 |
| (M₄) | | | | | | |
| M3007.4 | 9.1 | 1.4 | 63.3 | 13.7 | 5.5 | 12.7 |
| (M₅) | | | | | | |

| | | | | | | |
|---|---|---|---|---|---|---|
| *Sats=Total Saturate Content | | | | | | |

To determine the genetic relationship of the high palmitic mutation in M3007.4 to the low palmitic mutation in A144 (Example 1) crosses were made. The F₂ progeny were analyzed for fatty acid composition. The data presented in Table VIB shows the high palmitic group (C_{16.0} > 7.0%) makes up one-quarter of the total population analyzed. The high palmitic acid mutation was controlled by one single gene mutation.

**TABLE VIB Genetic Studies of M3007 X A144**

| | | Frequency | |
|---|---|---|---|
| Genotype | C_{16:0} Content(%) | Observed | Expected |
| p-p-/p-hp- | <7.0 | 151 | 142 |
| hp-hp- | >7.0 | 39 | 47 |

An additional M₃ line, W4773.7, contained 4.5% palmitic acid. Selfed progenies of this line, since designated A200.7 (ATCC 40816), continued to exceed the upper statistical threshold for high palmitic acid in both the M₄ and M₅ generations with palmitic acid levels of 6.3% and 6.0%, respectively. The fatty acid composition of this high palmitic acid mutant, which was stable to the M₇ generation under both field and greenhouse conditions, is summarized in Table VII.

**TABLE VII Fatty Acid Composition of a High Palmitic Acid Canola Line Produced by Seed Mutagenesis**

| | | | | Percent Fatty Acids | | |
|---|---|---|---|---|---|---|
| **Genotype** | C_{16:0} | C_{18:0} | C_{18.1} | C_{18:2} | C_{18:3} | Sats* |
| Westar | 3.9 | 1.9 | 67.5 | 17.6 | 7.4 | 7.0 |
| W4773.7 | 4.5 | 2.9 | 63.5 | 19.9 | 7.1 | 9.3 |
| (M₃) | | | | | | |
| M4773.7.7 | 6.3 | 2.6 | 59.3 | 20.5 | 5.6 | 10.8 |
| (M₄) | | | | | | |
| A200.7.7 | 6.0 | 1.9 | 60.2 | 20.4 | 7.3 | 9.4 |
| (M₅) | | | | | | |

| | | | | | | |
|---|---|---|---|---|---|---|
| *Sats=Total Saturate Content | | | | | | |

### EXAMPLE 5

### Selection of Low Stearic Acid Canola Lines

In the studies of Example 1, at the M₃ generation, 42 lines exceeded the lower statistical threshold for stearic acid (<1.4%). Line W14859.6 had 1.3% stearic acid. At the M₅ generation, its selfed progeny (M3052.1) continued to fall within the lower statistical threshold for C_{18:0} with 0.8% stearic acid. The fatty acid composition of this low stearic acid mutant, which was stable under both field and greenhouse conditions is summarized in Table VIII. In a single location replicated yield trial M3052.1 was not significantly different in yield from the parent cultivar Westar.

**TABLE VIII Fatty Acid Composition of a Low Stearic Acid Canola Line Produced by Seed Mutagenesis**

| | | | | Percent Fatty Acids | | |
|---|---|---|---|---|---|---|
| Genotype | C_{16:0} | C_{18.0} | C_{18:1} | C_{18:2} | C_{18:3} | Sats |
| Westar | 3.9 | 1.9 | 67.5 | 17.6 | 7.4 | 5.9 |
| W14859.6 | 5.3 | 1.3 | 56.1 | 23.7 | 9.6 | 7.5 |
| (M₃) | | | | | | |
| M3052.1 | 4.9 | 0.9 | 58.9 | 22.7 | 9.3 | 5.8 |
| (M₄) | | | | | | |
| M3052.6 | 4.4 | 0.8 | 62.1 | 21.2 | 7.9 | 5.2 |
| (M₅) | | | | | | |

To determine the genetic relationship of the low stearic acid mutation of M3052.1 to other fatty acid mutations it was crossed to the low palmitic acid mutation A144 (Example 1). Seed from over 300 dihaploid progeny were harvested and analyzed for fatty acid composition. The results are summarized in Table VIIIB. Independent segregation of the palmitic acid and stearic acid traits was observed. The low stearic acid mutation was genetically different from the low palmitic acid mutations found in A144 and M3094.

**TABLE VIIIB Genetic Studies of M3052 X A144**

| | | Frequency | |
|---|---|---|---|
| Genotype | C_{16:0} + C_{18:0} Content(%) | Observed | Expected |
| p-p-s-s- | <4.9% | 87 | 77 |
| p-p-s-s-/p+p+s-s- | 4.0%<X<5.6% | 152 | 154 |
| p+p+s+s+ | >5.6% | 70 | 77 |

An additional M₅ line, M3051.10, contained 0.9% and 1.1% stearic acid in the greenhouse and field respectively. A ten-seed analysis of this line showed the following fatty acid composition: C_{16:0} - 3.9%, C_{18:0} - 1.1%, C_{18:1} - 61.7%, C_{18:2} - 23.0%, C_{18:3} - 7.6%, FDA saturates - 5.0%, Total Saturates - 5.8%. In a single location replicated yield trial M3051.10 was not significantly different in yield from the parent cultivar Westar. M3051.10 was crossed to M3052.1 for allelism studies. Fatty acid composition of the F₂ seed showed the two lines to be allelic. The mutational events in M3051.10 and M3052.1 although different in origin were in the same gene.

An additional M₅ line, M3054.7, contained 1.0% and 1.3% stearic acid in the greenhouse and field respectively. A ten-seed analysis of this line showed the following fatty acid composition: C_{16:0} - 4.0%, C_{18:0} - 1.0%, C_{18:1} - 66.5%, C_{18:2} - 18:4%, C_{18:3} - 7.2%, saturates - 5.0%, Total Saturates - 6.1%. In a single location replicated yield trial M3054.7 was not significantly different in yield from the parent cultivar Westar. M3054.7 was crossed to M3052.1 for allelism studies. Fatty acid composition of the F₂ seed showed the two lines to be allelic. The mutational events in M3054.7, M3051.10 and M3052.1 although different in origin were in the same gene.

### EXAMPLE 6

### High Oleic Acid Canola Lines

In the studies of Example 1, at the M₃ generation, 31 lines exceeded the upper statistical threshold for oleic acid (≥ 71.0%). Line W7608.3 had 71.2% oleic acid. At the M₄ generation, its selfed progeny (W7608.3.5, since designated A129.5) continued to exceed the upper statistical threshold for C_{18.1} with 78.8% oleic acid. M₅ seed of five self-pollinated plants of line A129.5 (ATCC 40811) averaged 75.0% oleic acid. A single plant selection, A129.5.3 had 75.6% oleic acid. The fatty acid composition of this high oleic acid mutant, which was stable under both field and greenhouse conditions to the M, generation, is summarized in Table IX. This line also stably maintained its mutant fatty acid composition to the M, generation in field trials in multiple locations. Over all locations the self-pollinated plants (A129) averaged 78.3% oleic acid. The fatty acid composition of the A129 for each Idaho trial location are summarized in Table X. In multiple location replicated yield trials, A129 was not significantly different in yield from the parent cultivar Westar.

The canola oil of A129, after commercial processing, was found to have superior oxidative stability compared to Westar when measured by the Accelerated Oxygen Method (AOM), American Oil Chemists' Society Official Method Cd 12-57 for fat stability; Active Oxygen Method (revised 1989). The AOM of Westar was 18 AOM hours and for A129 was 30 AOM hours.

**TABLE IX Fatty Acid Composition of a High Oleic Acid Canola Line Produced by Seed Mutagenesis**

| | | | Percent Fatty Acids | | | |
|---|---|---|---|---|---|---|
| Genotype | C_{16:0} | C_{18:0} | C_{18:1} | C_{18:2} | C_{18:3} | Sats |
| Westar | 3.9 | 1.9 | 67.5 | 17.6 | 7.4 | 7.0 |
| W7608.3 | 3.9 | 2.4 | 71.2 | 12.7 | 6.1 | 7.6 |
| (M₃) | | | | | | |
| W7608.3.5 | 3.9 | 2.0 | 78.8 | 7.7 | 3.9 | 7.3 |
| (M₄) | | | | | | |
| A129.5.3 | 3.8 | 2.3 | 75.6 | 9.5 | 4.9 | 7.6 |
| (M₅) | | | | | | |

| | | | | | | |
|---|---|---|---|---|---|---|
| Sats=Total Saturate Content | | | | | | |

**TABLE X Fatty Acid Composition of a Mutant High Oleic Acid Line at Different Field Locations in Idaho**

| | | | Percent Fatty Acids | | | |
|---|---|---|---|---|---|---|
| Location | C_{16:0} | C_{18:0} | C_{18:1} | C_{18:2} | C_{18:3} | Sats |
| Burley | 3.3 | 2.1 | 77.5 | 8.1 | 6.0 | 6.5 |
| Tetonia | 3.5 | 3.4 | 77.8 | 6.5 | 4.7 | 8.5 |
| Lamont | 3.4 | 1.9 | 77.8 | 7.4 | 6.5 | 6.3 |
| Shelley | 3.3 | 2.6 | 80.0 | 5.7 | 4.5 | 7.7 |

| | | | | | | |
|---|---|---|---|---|---|---|
| Sats=Total Saturate Content | | | | | | |

The genetic relationship of the high oleic acid mutation A129 to other oleic desaturases was demonstrated in crosses made to commercial canola cultivars and a low linolenic acid mutation. A129 was crossed to the commercial cultivar Global (C_{16.0} - 4.5%, C_{18:0} - 1.5%, C_{18:1} - 62.9%, C_{18:2} - 20.0%, C_{18:3} - 7.3%). Approximately 200 F₂ individuals were analyzed for fatty acid composition. The results are summarized in Table XB. The segregation fit 1:2:1 ratio suggesting a single co-dominant gene controlled the inheritance of the high oleic acid phenotype.

**TABLE XB Genetic Studies of A129 X Global**

| | | Frequency | |
|---|---|---|---|
| Genotype | C_{18:0} Content(%) | Observed | Expected |
| od-od- | 77.3 | 43 | 47 |
| od-od+ | 71.7 | 106 | 94 |
| od+od+ | 66.1 | 49 | 47 |

A cross between A129 and IMC 01, a low linolenic acid variety (C_{16:0} - 4.1%, C_{18:0} - 1.9%, C_{18 1} - 66.4%, C_{18 2} - 18.1%, C_{18:3} - 5.7%), was made to determine the inheritance of the oleic acid desaturase and linoleic acid desaturase. In the F₁ hybrids both the oleic acid and linoleic acid desaturase genes approached the mid-parent values indicating a co-dominant gene actions. Fatty acid analysis of the F₂ individuals confirmed a 1:2:1:2:4:2:1:2:1 segregation of two independent, co-dominant genes (Table XC). A line was selected from the cross of A129 and IMC01 and designated as IMC130 (ATCC deposit no. 75446) as described in U.S. Patent Application No. 08/425,108, incorporated herein by reference.

**TABLE XC Genetic Studies of A129 X IMC 01**

| | | Frequency | |
|---|---|---|---|
| Genotype | Ratio | Observed | Expected |
| od-od-ld-ld- | 1 | 11 | 12 |
| od-od-ld-ld+ | 2 | 30 | 24 |
| od-od-ld+ld+ | 1 | 10 | 12 |
| od-od+ld-ld- | 2 | 25 | 24 |
| od-od+ld-ld+ | 4 | 54 | 47 |
| od-od+ld+ld+ | 2 | 18 | 24 |
| od+od+ld-ld- | 1 | 7 | 12 |
| od+od+ld-ld+ | 2 | 25 | 24 |
| od+od+ld+ld+ | 1 | 8 | 12 |

An additional high oleic acid line, designated A128.3, was also produced by the disclosed method. A 50-seed bulk analysis of this line showed the following fatty acid composition: C_{16.0} - 3.5%, C_{18:0} - 1.8%, C_{18:1} - 77.3%, C_{18:2} - 9.0%, C_{18:3} - 5.6%, FDA Sats - 5.3%, Total Sats - 6.4%. This line also stably maintained its mutant fatty acid composition to the M₇ generation. In multiple locations replicated yield trials, A128 was not significantly different in yield from the parent cultivar Westar.

A129 was crossed to A128.3 for allelism studies.

Fatty acid composition of the F₂ seed showed the two lines to be allelic. The mutational events in A129 and A128.3 although different in origin were in the same gene.

An additional high oleic acid line, designated M3028.-10 (ATCC 75026), was also produced by the disclosed method in Example 1. A 10-seed bulk analysis of this line showed the following fatty acid composition: C_{16:0} - 3.5%, C_{18:0} - 1.8%, C_{18:1} - 77.3%, C_{18:2} - 9.0%, C_{18:3} - 5.6%, FDA Saturates - 5.3%, Total Saturates - 6.4%. In a single location replicated yield trial M3028.10 was not significantly different in yield from the parent cultivar Westar.

### EXAMPLE 7

### Low Linoleic Acid Canola

In the studies of Example 1, at the M₃ generation, 80 lines exceeded the lower statistical threshold for linoleic acid (≤ 13.2%). Line W12638.8 had 9.4% linoleic acid. At the M₄ and M₅ generations, its selfed progenies [W12638.8, since designated A133.1 (ATCC 40812)] continued to exceed the statistical threshold for low C_{18:2} with linoleic acid levels of 10.2% and 8.4%, respectively. The fatty acid composition of this low linoleic acid mutant, which was stable to the M₇ generation under both field and greenhouse conditions, is summarized in Table XI. In multiple location replicated yield trials, A133 was not significantly different in yield from the parent cultivar Westar. An additional low linoleic acid line, designated M3062.8 (ATCC 75025), was also produced by the disclosed method. A 10-seed bulk analysis of this line showed the following fatty acid composition: C_{16:0} - 3.8%, C_{18:0} - 2.3%, C_{18:1} - 77.1%, C_{18:2} - 8.9%, C_{18:3} - 4.3%, FDA Sats-6.1%. This line has also stably maintained its mutant fatty acid composition in the field and greenhouse.

**TABLE XI Fatty Acid Composition of a Low Linoleic Acid Canola Line Produced by Seed Mutagenesis**

| | | | | Percent Fatty Acids | | |
|---|---|---|---|---|---|---|
| **Genotype** | C_{16:0} | C_{18:0} | C_{18:1} | C_{18:2} | C_{18:3} | Sats^{b} |
| Westar | 3.9 | 1.9 | 67.5 | 17.6 | 7.4 | 7.0 |
| W12638.8 | 3.9 | 2.3 | 75.0 | 9.4 | 6.1 | 7.5 |
| (M₃) | | | | | | |
| W12638.8.1 | 4.1 | 1.7 | 74.6 | 10.2 | 5.9 | 7.1 |
| (M₄) | | | | | | |
| A133.1.8 | 3.8 | 2.0 | 77.7 | 8.4 | 5.0 | 7.0 |
| (M₅) | | | | | | |

| | | | | | | |
|---|---|---|---|---|---|---|
| ^{a}Letter and numbers up to second decimal point indicate the plant line. Number after second decimal point indicates an individual plant. | | | | | | |
| ^{b}Sats=Total Saturate Content | | | | | | |

### EXAMPLE 8

### Low Linolenic and Linoleic Acid Canola

In the studies of Example 1, at the M₃ generation, 57 lines exceeded the lower statistical threshold for linolenic acid (≤ 5.3%). Line W14749.8 had 5.3% linolenic acid and 15.0% linoleic acid. At the M₄ and M₅ generations, its selfed progenies [W14749.8, since designated M3032 (ATCC 75021)] continued to exceed the statistical threshold for low C_{18.3} with linolenic acid levels of 2.7% and 2.3%, respectively, and for a low sum of linolenic and linoleic acids with totals of 11.8% and 12.5% respectively. The fatty acid composition of this low linolenic acid plus linoleic acid mutant, which was stable to the M₅ generation under both field and greenhouse conditions, is summarized in Table XII. In a single location replicated yield trial M3032 was not significantly different in yield from the parent cultivar (Westar).

**TABLE XII Fatty Acid Composition of a Low Linolenic Acid Canola Line Produced by Seed Mutagenesis**

| | | | Percent Fatty Acids | | | |
|---|---|---|---|---|---|---|
| **Genotype** | C_{16.0} | C_{18.0} | C_{18:1} | C_{18.2} | C_{18:3} | Sats |
| Westar | 3.9 | 1.9 | 67.5 | 17.6 | 7.4 | 7.0 |
| W14749.8 | 4.0 | 2.5 | 69.4 | 15.0 | 5.3 | 6.5 |
| (M₃) | | | | | | |
| M3032.8 | 3.9 | 2.4 | 77.9 | 9.1 | 2.7 | 6.4 |
| (M₄) | | | | | | |
| M3032.1 | 3.5 | 2.8 | 80.0 | 10.2 | 2.3 | 6.5 |
| (M₅) | | | | | | |

| | | | | | | |
|---|---|---|---|---|---|---|
| Sats=Total Saturate Content | | | | | | |

### EXAMPLE 9

The high oleic acid mutation of A129 was introduced into different genetic backgrounds by crossing and selecting for fatty acid and agronomic characteristics. A129 (now renamed IMC 129) was crossed to Legend, a commercial spring Brassica *napus* variety. Legend has the following fatty acid composition: C_{16.0} - 3.8%, C_{18:0} - 2.1%, C_{18:1} - 63.1%, C_{18:2} - 17.8%, C_{18:3} - 9.3%. The cross and progeny resulting from were coded as 89B60303.

The F₁ seed resulting from the cross was planted in the greenhouse and self-pollinated to produce F₂ seed. The F₂ seed was planted in the field for evaluation. Individual plants were selected in the field for agronomic characteristics. At maturity, the F₃ seed was harvested from each selected plant and analyzed for fatty acid composition.

Individuals which had fatty acid profiles similar to the high oleic acid parent (IMC 129) were advanced back to the field. Seeds (F₃) of selected individuals were planted in the field as selfing rows and in plots for preliminary yield and agronomic evaluations. At flowering the F₃ plants in the selfing rows were self-pollinated. At maturity the F₄ seed was harvested from individual plants to determine fatty acid composition. Yield of the individual selections was determined from the harvested plots.

Based on fatty acid composition of the individual plants and yield and agronomic characteristics of the plots F₄ lines were selected and advanced to the next generation in the greenhouse. Five plants from each selected line were self-pollinated. At maturity the F₅ seed was harvested from each and analyzed for fatty acid composition.

The F₅ line with the highest oleic fatty profile was advanced to the field as a selfing row. The remaining F₅ seed from the five plants was bulked together for planting the yield plots in the field. At flowering, the F₅ plants in each selfing-row were self-pollinated. At maturity the F₆ self-pollinated seed was harvest from the selfing row to determine fatty acid composition and select for the high oleic acid trait. Yield of the individual selections was determined from the harvested plots.

Fifteen F₆ lines having the high oleic fatty profile of IMC 129 and the desired agronomic characteristics were advanced to the greenhouse to increase seed for field trialing. At flowering the F₆ plants were self-pollinated. At maturity the F, seed was harvested and analyzed for fatty acid composition. Three F₇ seed lines which had fatty acid profiles most similar to IMC 129 (Table XIII) were selected and planted in the field as selfing rows, the remaining seed was bulked together for yield trialing. The high oleic fatty acid profile of IMC 129 was maintained through seven generations of selection for fatty acid and agronomic traits in an agronomic background of *Brassica* napus which was different from the parental lines. Thus, the genetic trait from IMC 129 for high oleic acid can be used in the development of new high oleic *Brassica napus* varieties.

**TABLE XIII Fatty Acid Composition of Advanced Breeding Generation with High Oleic Acid Trait (IMC 129 X Legend)**

| | | Fatty Acid Composition(%) | | | |
|---|---|---|---|---|---|
| F₇ Selections of 89B60303 | C_{16:0} | C_{18:0} | C_{18:1} | C_{18:2} | C_{18:3} |
| 93.06194 | 3.8 | 1.6 | 78.3 | 7.7 | 4.4 |
| 93.06196 | 4.0 | 2.8 | 77.3 | 6.8 | 3.4 |
| 93.06198 | 3.7 | 2.2 | 78.0 | 7.4 | 4.2 |

The high oleic acid trait of IMC 129 was also introduced into a different genetic background by combining crossing and selection methods with the generation of dihaploid populations from the microspores of the F₁ hybrids. IMC 129 was crossed to Hyola 41, a commercial spring *Brassica* napus variety. Hyola 41 has the following fatty acid composition: C_{16:0} - 3.8%, C_{18:0} - 2.7%, C_{18:1} - 64.9%, C_{18:2} - 16.2%, C_{18:3} - 9.1%. The cross and progeny resulting from the cross were labeled 90DU.146.

The F₁ seed was planted from the cross and a dihaploid (DH₁) population was made from the F₁ microspores using standard procedures for *Brassica napus.* Each DH₁ plant was self-pollinated at flowering to produce DH₁ seed. At maturity the DH₁ seed was harvested and analyzed for fatty acid composition. DH₁ individuals which expressed the high oleic fatty acid profit of IMC 129 were advanced to the next generation in the greenhouse. For each individual selected five DH₁ seeds were planted. At flowering the DH₂ plants were self-pollinated. At maturity the DH₂ seed was harvested and analyzed for fatty acid composition. The DH₂ seed which was similar in fatty acid composition to the IMC 129 parent was advanced to the field as a selfing row. The remaining DH₂ seed of that group was bulked and planted in plots to determine yield and agronomic characteristics of the line. At flowering individual DH₃ plants in the selfing row were self-pollinated. At maturity the DH₃ seed was harvested from the individual plants to determine fatty acid composition. Yield of the selections was determined from the harvested plots. Based on fatty acid composition, yield and agronomic characteristics selections were advanced to the next generation in the greenhouse. The DH₄ seed produced in the greenhouse by self-pollination was analyzed for fatty acid composition. Individuals which were similar to the fatty acid composition of the IMC 129 parent were advanced to the field to test for fatty acid stability and yield evaluation. The harvested DH₅ seed from six locations maintained the fatty acid profile of the IMC 129 parent (Table XIV).

**TABLE XIV Fatty Acid Composition of Advanced Dihaploid Breeding Generation with High Oleic Acid Trait (IMC 129 X Hyola41)**

| | | Fatty Acid Composition(%) | | | |
|---|---|---|---|---|---|
| DH5 of 90DU.146 at Multiple Locations | C_{16:0} | C_{18:0} | C_{18:1} | C_{18:2} | C_{18:3} |
| Aberdeen | 3.7 | 2.6 | 75.4 | 8.1 | 7.2 |
| Blackfoot | 3.3 | 2.4 | 75.5 | 8.8 | 7.5 |
| Idaho Falls | 3.7 | 3.1 | 75.0 | 7.5 | 8.1 |
| Rexberg | 3.9 | 3.7 | 75.3 | 7.0 | 6.5 |
| Swan Valley | 3.5 | 3.4 | 74.5 | 7.0 | 7.3 |
| Lamont | 3.9 | 2.8 | 72.0 | 10.1 | 8.4 |

### EXAMPLE 10

### Canola Lines 0508 and 04275

Seeds of the *B. napus* line IMC-129 were mutagenized with methyl N-nitrosoguanidine (MNNG). The MNNG treatment consisted of three parts: pre-soak, mutagen application, and wash. A 0.05M Sorenson's phosphate buffer was used to maintain pre-soak and mutagen treatment pH at 6.1. Two hundred seeds were treated at one time on filter paper (Whatman #3M) in a petri dish (100mm x 15mm). The seeds were pre-soaked in 15 mls of 0.05M Sorenson's buffer, pH 6.1, under continued agitation for two hours. At the end of the pre-soak period, the buffer was removed from the plate.

A 10mM concentration of MNNG in 0.05M Sorenson's buffer, pH 6.1, was prepared prior to use. Fifteen ml of 10m MNNG was added to the seeds in each plate. The seeds were incubated at 22°C±3°C in the dark under constant agitation for four (4) hours. At the end of the incubation period, the mutagen solution was removed.

The seeds were washed with three changes of distilled water at 10 minute intervals. The fourth wash was for thirty minutes. This treatment regime produced an LD60 population.

Treated seeds were planted in standard greenhouse potting soil and placed into an environmentally controlled greenhouse. The plants were grown under sixteen hours of light. At flowering, the racemes were bagged to produce selfed seed. At maturity, the M2 seed was harvested. Each M2 line was given an identifying number. The entire MNNG-treated seed population was designated as the Q series.

Harvested M2 seeds was planted in the greenhouse. The growth conditions were maintained as previously described. The racemes were bagged at flowering for selfing. At maturity, the selfed M3 seed was harvested and analyzed for fatty acid composition. For each M3 seed line, approximately 10-15 seeds were analyzed in bulk as described in Example 1.

High oleic-low linoleic M3 lines were selected from the M3 population using a cutoff of >82% oleic acid and <5.0% linoleic. From the first 1600 M3 lines screened for fatty acid composition, Q508 was identified. The Q508 M3 generation was advanced to the M4 generation in the greenhouse. Table XV shows the fatty acid composition of Q508 and IMC 129. The M4 selfed seed maintained the selected high oleic-low linoleic acid phenotype (Table XVI).

**TABLE XV Fatty Acid Composition of A129 and High Oleic Acid M3 Mutant O508**

| | | | | | |
|---|---|---|---|---|---|
| Line # | 16:0 | 18:0 | 18:1 | 18:2 | 18:3 |
| A129' | 4.0 | 2.4 | 77.7 | 7.8 | 4.2 |
| O508 | 3.9 | 2.1 | 84.9 | 2.4 | 2.9 |

| | | | | | |
|---|---|---|---|---|---|
| *Fatty acid composition of A129 is the average of 50 self-pollinated plants grown with the M3 population | | | | | |

M₄ generation Q508 plants had poor agronomic qualities in the field compared to Westar. Typical plants were slow growing relative to Westar, lacked early vegetative vigor, were short in stature, tended to be chlorotic and had short pods. The yield of Q508 was very low compared to Westar.

The M₄ generation Q508 plants in the greenhouse tended to be reduced in vigor compared to Westar. However, Q508 yields in the greenhouse were greater than Q508 yields in the field.

**TABLE XVI Fatty Acid Composition of Seed Oil from Greenhouse-Grown O508, IMC 129 and Westar.**

| | | | | | | |
|---|---|---|---|---|---|---|
| Line | 16:0 | 18:0 | 18:1 | 18:2 | 18:3 | FDA Sats |
| IMC 129^{a} | 4.0 | 2.4 | 77.7 | 7.8 | 4.2 | 6.4 |
| Westar^{b} | 3.9 | 1.9 | 67.5 | 17.6 | 7.4 | >5.8 |
| Q508^{c} | 3.9 | 2.1 | 84.9 | 2.4 | 2.9 | 6.0 |

| | | | | | | |
|---|---|---|---|---|---|---|
| ^{a}Average of 50 self-pollinated plants | | | | | | |
| ^{b}Data from Example 1 | | | | | | |
| ^{c}Average of 50 self-pollinated plants | | | | | | |

Nine other M4 high-oleic low-linoleic lines were also identified: Q3603, Q3733, Q4249, Q6284, Q6601, Q6761, Q7415, Q4275, and Q6676. Some of these lines had good agronomic characteristics and an elevated oleic acid level in seeds of about 80% to about 84%.

Q4275 was crossed to the variety Cyclone. After selfing for seven generations, mature seed was harvested from 93GS34-179, a progeny line of the Q4275 Cyclone cross. Referring to Table XVII, fatty acid composition of a bulk seed sample shows that 93GS34 retained the seed fatty acid composition of Q4275. 93GS34-179 also maintained agronomically desirable characteristics.

After more than seven generations of selfing of Q4275, plants of Q4275, IMC 129 and 93GS34 were field grown during the summer season. The selections were tested in 4 replicated plots (5 feet X 20 feet) in a randomized block design. Plants were open pollinated. No selfed seed was produced. Each plot was harvested at maturity, and a sample of the bulk harvested seed from each line was analyzed for fatty acid composition as described above. The fatty acid compositions of the selected lines are shown in Table XVII.

**Table XVII Fatty Acid Composition of Field Grown IMC 129, Q4275 and 93GS34 Seeds**

| Line | **Fatty Acid Composition (%)** | | | | | |
|---|---|---|---|---|---|---|
| | C_{16:0} | C_{18:0} | C_{18:1} | C_{18:2} | C_{18:3} | FDA Sats |
| IMC 129 | 3.3 | 2.4 | 76.7 | 8.7 | 5.2 | 5.7 |
| Q4275 | 3.7 | 3.1 | 82.1 | 4.0 | 3.5 | 6.8 |
| 93GS34-179 | 2.6 | 2.7 | 85.0 | 2.8 | 3.3 | 5.3 |

The results shown in Table XVII show that Q4275 maintained the selected high oleic - low linoleic acid phenotype under field conditions. The agronomic characteristics of Q4275 plants were superior to those of Q508.

M₄ generation Q508 plants were crossed to a dihaploid selection of Westar, with Westar serving as the female parent. The resulting F1 seed was termed the 92EF population. About 126 F1 individuals that appeared to have better agronomic characteristics than the Q508 parent were selected for selfing. A portion of the F₂ seed from such individuals was replanted in the field. Each F2 plant was selfed and a portion of the resulting F3 seed was analyzed for fatty acid composition. The content of oleic acid in F₃ seed ranged from 59 to 79%. No high oleic (>80%) individuals were recovered with good agronomic type.

A portion of the F₂ seed of the 92EF population was planted in the greenhouse to analyze the genetics of the Q508 line. F₃ seed was analyzed from 380 F2 individuals. The C_{18.1} levels of F₃ seed from the greenhouse experiment is depicted in Figure 1. The data were tested against the hypothesis that Q508 contains two mutant genes that are semi-dominant and additive: the original IMC 129 mutation as well as one additional mutation. The hypothesis also assumes that homozygous Q508 has greater than 85% oleic acid and homozygous Westar has 62-67% oleic acid. The possible genotypes at each gene in a cross of Q508 by Westar may be designated as:
AA = Westar Fad2^{a}
BB = Westar Fad2^{b}
aa = Q508 Fad2^{a-}
bb = Q508 Fad2^{b-}
Assuming independent segregation, a 1:4:6:4:1 ratio of phenotypes is expected. The phenotypes of heterozygous plants are assumed to be indistinguishable and, thus, the data were tested for fit to a 1:14:1 ratio of homozygous Westar: heterozygous plants: homozygous Q508.

| Phenotypic Ratio | # of Westar Alleles | Genotype |
|---|---|---|
| 1 | 4 | AABB(Westar) |
| 4 | 3 | AABb,AaBB,AABb,AaBB |
| 6 | 2 | AaBb,AAbb,AaBb,AaBb,aaBB,AaBb |
| 4 | 1 | Aabb,aaBb,Aabb,aaBb |
| 1 | 0 | aabb (Q508) |

Using Chi-square analysis, the oleic acid data fit a 1:14:1 ratio. It was concluded that Q508 differs from Westar by two major genes that are semi-dominant and additive and that segregate independently. By comparison, the genotype of IMC 129 is aaBB.

The fatty acid composition of representative F3 individuals having greater than 85% oleic acid in seed oil is shown in Table XVIII. The levels of saturated fatty acids are seen to be decreased in such plants, compared to Westar.

**TABLE XVIII 92EF F₃ Individuals with >85% C_{18:1} in Seed Oil**

| F3 Plant Identifier | Fatty Acid Composition (%) | | | | | |
|---|---|---|---|---|---|---|
| | C16:0 | C18:0 | C18:1 | C18:2 | C18:3 | FDASA |
| +38068 | 3.401 | 1.582 | 85.452 | 2.134 | 3.615 | 4.983 |
| +38156 | 3.388 | 1.379 | 85.434 | 2.143 | 3.701 | 4.767 |
| +38171 | 3.588 | 1.511 | 85.289 | 2.367 | 3.425 | 5.099 |
| +38181 | 3.75 | 1.16 | 85.312 | 2.968 | 3.819 | 4.977 |
| +38182 | 3.529 | 0.985 | 85.905 | 2.614 | 3.926 | 4.56 |
| +38191 | 3.364 | 1.039 | 85.737 | 2.869 | 4.039 | 4.459 |
| +38196 | 3.557 | 1.182 | 85.054 | 2.962 | 4.252 | 4.739 |
| +38202 | 3.554 | 1.105 | 86.091 | 2.651 | 3.721 | 4.713 |
| +38220 | 3.093 | 1.16 | 86.421 | 1.931 | 3.514 | 4.314 |
| +38236 | 3.308 | 1.349 | 85.425 | 2.37 | 3.605 | 4.718 |
| +38408 | 3.617 | 1.607 | 85.34 | 2.33 | 3.562 | 5.224 |
| +38427 | 3.494 | 1.454 | 85.924 | 2.206 | 3.289 | 4.948 |
| +38533 | 3.64 | 1.319 | 85.962 | 2.715 | 3.516 | 4.959 |

### EXAMPLE 11

### Leaf and Root Fatty Acid Profiles of Canola Lines IMC-129, Q508, and Westar

Plants of Q508, IMC 129 and Westar were grown in the greenhouse. Mature leaves, primary expanding leaves, petioles and roots were harvested at the 6-8 leaf stage, frozen in liquid nitrogen and stored at -70°C. Lipid extracts were analyzed by GLC as described in Example 1. The fatty acid profile data are shown in Table XIX.

The data in Table XIX indicate that total leaf lipids in Q508 are higher in C_{18:1} content than the C_{18:2} plus C_{18:3} content. The reverse is true for Westar and IMC 129. The difference in total leaf lipids between Q508 and IMC 129 is consistent with the hypothesis that a second Fad2 gene is mutated in Q508.

The C_{16:3} content in the total lipid fraction was about the same for all three lines, suggesting that the plastid FadC gene product was not affected by the Q508 mutations. To confirm that the FadC gene was not mutated, chloroplast lipids were separated and analyzed. No changes in chloroplast C_{16:1}, C_{16:2} or C_{16:3} fatty acids were detected in the three lines. The similarity in plastid leaf lipids among Q508, Westar and IMC 129 is consistent with the hypothesis that the second mutation in Q508 affects a microsomal Fad2 gene and not a plastid FadC gene.

**TABLE XIX**

| | MATURE LEAF | | | EXPANDING LEAF | | | PETIOLE | | | ROOT | | |
|---|---|---|---|---|---|---|---|---|---|---|---|---|
| | West | 129 | 3Q508 | West. | 129 | 3Q508 | West. | 129 | 3Q508 | West. | 129 | 3Q508 |
| 16:0 | 12.1 | 11.9 | 10.1 | 16 4 | 16.1 | 11 3 | 21.7 | 23.5 | 11.9 | 21.1 | 21 9 | 12.0 |
| 16 1 | 0.8 | 0.6 | 1.1 | 0.7 | 0 6 | 1.1 | 1 0 | 1.3 | 1 4 | - | - | - |
| 16:2 | 2.3 | 2.2 | 2.0 | 2.8 | 3.1 | 2.8 | 1.8 | 2.2 | 1.8 | - | - | - |
| 16:3 | 14.7 | 15.0 | 14.0 | 6.3 | 5.4 | 6 9 | 5.7 | 4.6 | 5.7 | - | - | - |
| 18:0 | 2.2 | 1.6 | 1.2 | 2 5 | 2.8 | 1.5 | 3.7 | 4.0 | 1.6 | 3.6 | 2.9 | 2.5 |
| 18:1 | 2.8 | 4.9 | 16.7 | 3.8 | 8.3 | 38.0 | 4 9 | 12.9 | 46.9 | 3.5 | 6.1 | 68.8 |
| 18:2 | 12.6 | 11.5 | 6.8 | 13.3 | 13.8 | 4.9 | 20.7 | 18.3 | 5.2 | 28.0 | 30.4 | 4.4 |
| 18:3 | 50.6 | 50.3 | 46.0 | 54.2 | 50.0 | 33.5 | 40.4 | 33.2 | 25.3 | 43.8 | 38.7 | 12.3 |

### EXAMPLE 12

### Sequences of Mutant and Wild-Type Delta-12 Fatty Acid Desaturases from B. napus

Primers specific for the FAD2 structural gene were used to clone the entire open reading frame (ORF) of the D and F 12-DES genes by reverse transcriptase polymerase chain reaction (RT-PCR). RNA from seeds of IMC 129, Q508 and Westar plants was isolated by standard methods and was used as template. The RT-amplified fragments were used for nucleotide sequence determination. The DNA sequence of each gene from each line was determined from both strands by standard dideoxy sequencing methods.

Sequence analysis revealed a G to A transversion at nucleotide 316 (from the translation initiation codon) of the D gene in both IMC 129 (SEQ ID NO:3) and Q508, compared to the sequence of Westar (SEQ ID NO:1). The transversion changes the codon at this position from GAG to AAG and results in a non-conservative substitution of glutamic acid, an acidic residue, for lysine a basic residue. The presence of the same mutation in both lines was expected since the Q508 line was derived from IMC 129. The same base change was also detected in Q508 and IMC 129 when RNA from leaf tissue was used as template.

The G to A mutation at nucleotide 316 was confirmed by sequencing several independent clones containing fragments amplified directly from genomic DNA of IMC 129 and Westar. These results eliminated the possibility of a rare mutation introduced during reverse transcription and PCR in the RT-PCR protocol. It was concluded that the IMC 129 mutant is due to a single base transversion at nucleotide 316 in the coding region of the D gene of rapeseed microsomal delta 12-desaturase.

A single base transition from T to A at nucleotide 515 of the F gene was detected in Q508 compared to the Westar.sequence- The mutation changes the codon at this position from CTC to CAC, resulting in the non-conservative substitution of a polar residue, histidine, for a non-polar residue, leucine, for in the resulting gene product. No mutations were found in the F gene sequence of IMC 129 compared to the F gene sequence of Westar.

These data support the conclusion that a mutation in a delta-12 desaturase gene sequence results in alterations in the fatty acid profile of plants containing such a mutated gene. Moreover, the data show that when a plant line or species contains two delta-12 desaturase loci, the fatty acid profile of an individual having two mutated loci differs from the fatty acid profile of an individual having one mutated locus.

The mutation in the D gene of IMC 129 and Q508 mapped to a region having a conserved amino acid motif (His-Xaa-Xaa-Xaa-His) found in cloned delta-12 and delta-15 membrane bound-desaturases (Table XX).

**Table XX Alignment of Amino Acid Sequences of Cloned Canola Membrane Bound-Desaturases**

| Desaturase Gene | Sequence^{a} | Position |
|---|---|---|
| Canola-fad2-D (mutant) | AHKCGH | 109-114 |
| Canola-Fad2-D | AHECGH | 109-114 |
| Canola-Fad2-F | AHECGH | 109-114 |
| Canola-FadC | GHDCAH | 170-175 |
| | | |
| Canola-fad3 (mutant) | GHKCGH | 94-99 |
| Canola-Fad3 | GHDCGH | 94-99 |
| Canola-FadD | GHDCGH | 125-130 |

| | | |
|---|---|---|
| (FadD = Plastid delta 15, Fad3 = Microsomal delta-15), | | |
| (FadC = Plastid delta-12, Fad2 = Microsomal delta-12) | | |
| ^{a} One letter amino acid code; conservative substitutions are underlined; non-conservative substitutions are in bold. | | |

### EXAMPLE 13

### Transcription and Translation of Microsomal Delta-12 Fatty Acid Desaturases

Transcription in vivo was analyzed by RT-PCR analysis of stage II and stage III developing seeds and leaf tissue. The primers used to specifically amplify 12-DES F gene RNA from the indicated tissues were sense primer 5'-GGATATGATGATGGTGAAAGA-3' and antisense primer 5'-TCTTTCACCATCATCATATCC-3'. The primers used to specifically amplify 12-DES D gene RNA from the indicated tissues were sense primer 5'-GTTATGAAGCAAAGAAGAAAC-3' and antisense primer 5'-GTTTCTTCTTTGCTTCATAAC-3'. The results indicated that mRNA of both the D and F gene was expressed in seed and leaf tissues of IMC 129, Q508 and wild type Westar plants.

*In vitro* transcription and translation analysis showed that a peptide of about 46 kD was made. This is the expected size of both the D gene product and the F gene product, based on sum of the deduced amino acid sequence of each gene and the cotranslational addition of a microsomal membrane peptide.

These results rule out the possibility that nonsense or frameshift mutations, resulting in a truncated polypeptide gene product, are present in either the mutant D gene or the mutant F gene. The data, in conjunction with the data of Example 12, support the conclusion that the mutations in Q508 and IMC 129 are in delta-12 fatty acid desaturase structural genes encoding desaturase enzymes, rather than in regulatory genes.

### EXAMPLE 14

### Development of Gene-Specific PCR Markers

Based on the single base change in the mutant D gene of IMC 129 described in above, two 5' PCR primers were designed. The nucleotide sequence of the primers differed only in the base (G for Westar and A for IMC 129) at the 3' end. The primers allow one to distinguish between mutant fad2-D and wild-type Fad2-D alleles in a DNA-based PCR assay. Since there is only a single base difference in the 5' PCR primers, the PCR assay is very sensitive to the PCR conditions such as annealing temperature, cycle number, amount, and purity of DNA templates used. Assay conditions have been established that distinguish between the mutant gene and the wild type gene using genomic DNA from IMC 129 and wild type plants as templates. Conditions may be further optimized by varying PCR parameters, particularly with variable crude DNA samples. A PCR assay distinguishing the single base mutation in IMC 129 from the wild type gene along with fatty acid composition analysis provides a means to simplify segregation and selection analysis of genetic crosses involving plants having a delta-12 fatty acid desaturase mutation.

### EXAMPLE 15

### Transformation with Mutant and Wild Type Fad3 Genes

*B. napus* cultivar Westar was transformed with mutant and wild type Fad3 genes to demonstrate that the mutant Fad3 gene for canola cytoplasmic linoleic desaturase 15-DES is nonfunctional. Transformation and regeneration were performed using disarmed *Agrobacterium tumefaciens* essentially following the procedure described in WO 94/11516.

Two disarmed Agrobacterium strains were engineered, each containing a Ti plasmid having the appropriate gene linked to a seed-specific promoter and a corresponding termination sequence. The first plasmid, pIMC110, was prepared by inserting into a disarmed Ti vector the full length wild type Fad3 gene in sense orientation (nucleotides 208 to 1336 of SEQ ID 6 in WO 93/11245), flanked by a napin promoter sequence positioned 5' to the Fad3 gene and a napin termination sequence positioned 3' to the Fad3 gene. The rapeseed napin promoter is described in EP 0255378.

The second plasmid, pIMC205, was prepared by inserting a mutated Fad3 gene in sense orientation into a disarmed Ti vector. The mutant sequence contained mutations at nucleotides 411 and 413 of the microsomal Fad3 gene described in WO93/11245, thus changing the sequence for codon 96 from GAC to AAG. The amino acid at codon 96 of the gene product was thereby changed from aspartic acid to lysine. See Table XX. A bean (*Phaseolus vulgaris)* phaseolin (7S seed storage protein) promoter fragment of 495 base pairs, starting with 5'-TGGTCTTTTGGT-3', was placed 5' to the mutant Fad3 gene and a phaseolin termination sequence was placed 3' to the mutant Fad3 gene. The phaseolin sequence is described in Doyle et al., (1986) J. Biol. Chem. 261:9228-9238) and Slightom et al., (1983) Proc. Natl. Acad. Sci. USA 80:1897-1901.

The appropriate plasmids were engineered and transferred separately to *Agrobacterium* strain LBA4404. Each engineered strain was used to infect 5 mm segments of hypocotyl explants from Westar seeds by cocultivation. Infected hypocotyls were transferred to callus medium and, subsequently, to regeneration medium. Once discernable stems formed from the callus, shoots were excised and transferred to elongation medium. The elongated shoots were cut, dipped in Rootone™, rooted on an agar medium and transplanted to potting soil to obtain fertile T1 plants. T2 seeds were obtained by selfing the resulting T1 plants.

Fatty acid analysis of T2 seeds was carried out as described above. The results are summarized in Table XXI. of the 40 transformants obtained using the pIMC110 plasmid, 17 plants demonstrated wild type fatty acid profiles and 16 demonstrated overexpression. A proportion of the transformants are expected to display an overexpression phenotype when a functioning gene is transformed in sense orientation into plants.

Of the 307 transformed plants having the pIMC205 gene, none exhibited a fatty acid composition indicative of overexpression. This result indicates that the mutant fad3 gene product is non-functional, since some of the transformants would have exhibited an overexpression phenotype if the gene product were functional.

**Table XXI Overexpression and Co-suppression Events in Westar Populations Transformed with pIMC205 or pIMC110.**

| Construct | Number of Transformants | α-Linolenic Acid Range(%) | Overexpression Events (>10% linolenic) | Co-Suppression Events (<4.0% linolenic) | Wild Type Events |
|---|---|---|---|---|---|
| pIMC110 | 40 | 2.4 - 20.6 | 16 | 7 | 17 |
| pIMC205 | 307 | 4.6 - 10.4 | 0 | 0 | 307 |

Fatty acid compositions of representative transformed plants are presented in Table XXII. Lines 652-09 and 663-40 are representative of plants containing pIMC110 and exhibiting an overexpression and a co-suppression phenotype, respectively. Line 205-284 is representative of plants containing pIMC205 and having the mutant fad3 gene.

**Table XXII Fatty Acid Composition of T2 Seed From Westar Transformed With pIMC205 or pIMC110.**

| Line | Fatty Acid Composition (%) | | | | |
|---|---|---|---|---|---|
| | C16:0 | C18.0 | C18:1 | C18:2 | C18:3 |
| 652-09 pIMC110 overexpression | 4.7 | 3.3 | 65.6 | 8.1 | 14.8 |
| 663-40 pIMC110 co-suppression | 4.9 | 2.1 | 62.5 | 23.2 | 3.6 |
| 205-284 pIMC205 | 3.7 | 1.8 | 68.8 | 15.9 | 6.7 |

To the extent not already indicated, it will be understood by those of ordinary skill in the art that any one of the various specific embodiments herein described and illustrated may be further modified to incorporate features shown in other of the specific embodiments.

The foregoing detailed description has been provided for a better understanding of the invention only and no unnecessary limitation should be understood therefrom as some modifications will be apparent to those skilled in the art without deviating from the spirit and scope of the appended claims.

## Claims

1. An isolated nucleic acid fragment comprising a full-length coding sequence of a *Brassicaceae* or *Helianthus* delta-12 fatty acid desaturase gene, said coding sequence having at least one mutation in a region of said desaturase gene encoding a His-Glu-Cys-Gly-His amino acid motif, wherein said at least one mutation in said motif comprises a codon encoding Lys in place of the codon encoding Glu, and wherein said at least one mutation renders the product of said desaturase gene non-functional.

2. The nucleic add fragment of claim 1, wherein said mutant desaturase gene encodes a microsomal gene product.

3. The nucleic acid fragment of claim 1, wherein said mutant desaturase gene is from a *Brassica napus* plant.

4. The nucleic acid fragment of claim 1, wherein said gene is the D form of a *Brassicaccae* microsomal gene.

5. A plant of the *Brassicaceae* or *Helianthus* families other than *Brassica napus*, said plant containing a full-length coding sequence of a delta-12 fatty acid desaturase gene, said coding sequence having at least one mutation in a region encoding a His-Glu-Cys-Gly-His amino acid motif, wherein said at least one mutation in said motif comprises a codon encoding Lys in place of the codon encoding Glu, and wherein said mutation confers an attered fatty acid composition in seeds of said plant.

6. The plant of claim 5, wherein said gene is from a *Brassica napus* plant.

7. The plant of claim 5. wherein said plant is a *Brassica rapa* plant.

8. An isolated nucleic acid fragment comprising a full-length coding sequence of a *Brassicaceae* delta-15 fatty acid desaturase gene, said coding sequence having at least one mutation in a region of said desaturase gene encoding a His-Asp-Cys-Gly-His amino acid motif, wherein said at least one mutation in said motif comprises a codon encoding Lys in place of the codon encoding Asp, and wherein said at least one mutation renders the product of said desaturase gene non-functional.

9. The nucleic acid fragment of claim 8, wherein said mutant desaturase gene is from a *Brassica nopus* plant.

10. A *Brassicaceae* plant containing a full-length coding sequence of a delta-15 fatty acid desaturase gene having at least one mutation in a region encoding a His-Asp-Cys-Gly-His amino acid motif, wherein said at least one mutation in said motif comprises a codon encoding Lys in place of the codon encoding Asp, and wherein said mutation confers an altered fatty acid composition in seeds of said plant.

11. The plant of claim 10, wherein said mutant desaturase gene is from a *Brassica napus* plant.

12. The plant of claim 10, wherein said plant is a *Brassica napus* plant.

13. A *Brassicaceae* plant containing:
a) a full-lengfh coding sequence of a delta-12 fatty acid desaturase gene, said coding sequence having at least one mutation in a region encoding a His-Glu-Cys-Gly-His amino acid motif, wherein said at least one mutation in said motif comprises a codon encoding Lys in place of the codon encoding Glu;
b) a full length coding sequence of a delta-15 fatty acid desaturase gene having at least one mutation, said at least one delta-15 gene mutation in a region encoding a His-Xaa-Xaa-Xaa-His amino add motif, said delta-12 gene mutation and said delta-15 gene mutation conferring an altered fatty acid composition in seeds of said plant.

14. A method for producing a *Brassicaceae* or *Helianthus* plant line, comprising the steps of:
a) inducing mutagenesis in cells of a starting variety of a *Brassicaceae* or *Helianthus* species;
b) obtaining one or more progeny plants from said cells;
c) identifying at least one of said progeny plants that contains a delta-12 fatty acid desaturase gene having at least one mutation in a region encoding a His-Glu-Cys-Gly-His amino acid motif; wherein said at least one mutation in said motif comprises a codon encoding Lys in place of the codon encoding Glu, and wherein said at least one mutation renders the product of said delta-12 desaturase gene non-functional; and
d) producing said plant line from said at least one progeny plant by self-or cross-pollination, said plant line having said at least one delta-12 gene mutation.

15. The method of claim 14, wherein said plant line produces seeds yielding an oil having a stabilized linoleic acid content from about 2% to about 12%.

16. The method of claim 14, further comprising the steps of:
e) inducing mutagenesis in cells of said plant line;
f) obtaining one or more progeny plants from said plant line cells;
g) identifying at least one of said plant line progeny plants that contains a delta-15 fatty acid desaturase gene having at least one delta-15 gene mutation in a region encoding a His-Xaa-Xaa-Xaa-His amino acid motif. wherein said delta-15 gene mutation renders the product of said delta-15 desaturase non-functional;
h) producing a second plant line from said at least one plant line progeny plant by self- or cross-pollination, said second plant line having said at least one delta-12 gene mutation and said at least one delta-15 gene mutation.

17. The method of claim 14, wherein said starting variety is a *Brassica napus* variety.

18. The method of claim 17, wherein said mutation is in a first form of delta-12 fatty add desaturase.

19. The method of claim 18, further comprising the step of crossing a plant of said plant line to a plant having a mutation in a second form of delta-12 fatty acid desaturase.

20. The method of claim 19, wherein said second mutation is in a region other than a region encoding a His-Xaa-Xaa-Xaa-His amino acid motif.

21. The method of claim 17, further comprising the steps of:
e) inducing mutagenesis In cells of said plant line;
f) obtaining one or more progeny plants from said plant line cells;
g) identifying at least one of said plant line progeny plants that contains a second delta-12 fatty acid desaturase gene having at least one mutation, said second gene mutation in a region other than a region encoding a His-Xaa-Xaa-Xaa-His amino acid motif; and
h) producing a second plant line from said at least one plant line progeny plant by self- or cross-pollination, said second plant line having said first delta-12 gene mutation and said second delta-12 gene mutation.

22. A method for producing a *Brassicaceae* plant line, comprising the steps of:
a) inducing mutagenesis in cells of a starting variety of a *Brassicaceas* species;
b) obtaining one or more progeny plants from said cells;
c) identifying at least one of said progeny plants that contains a delta-15 fatty acid desaturase gene having at least one mutation in a region encoding a His-Asp-Cys-Gly-His amino acid motif; wherein said at least one mutation in said motif comprises a codon encoding Lys in place of the codon encoding Asp, and wherein said at least one mutation renders the product of said delta-15 desaturase gene non-functional; and
d) producing said plant line from said at least one progeny plant by self-or cross-pollination, said plant line having said at least one delta-15 gene mutation.

23. The method of claim 21 or claim 22. wherein said identifying step comprises a technique selected from the group consisting of PCR, 3SR and direct polynucleotide sequencing.

## Patentansprüche

1. Isoliertes Nucleinsäurefragment, das eine vollständige Kodierungssequenz eines Delta-12-Fettsäure-Desaturase-Genes von *Brassicaceae* oder *Helianthus* aufweist, wobei die Kodierungssequenz zumindest eine Mutation in einem Bereich des Desaturase-Genes aufweist, der für ein His-Glu-Cys-Gly-His-Aminosäuremotiv kodiert, wobei die zumindest eine Mutation in diesem Motiv ein Codon aufweist, das für Lys kodiert, anstelle des Codons, das für Glu kodiert, und wobei die zumindest eine Mutation das Produkt des Desaturase-Genes nicht-funktionell macht.

2. Nucleinsäurefragment nach Anspruch 1, wobei das mutierte Desaturase-Gen für ein mikrosomales Genprodukt kodiert.

3. Nucleinsäurefragment nach Anspruch 1, wobei das mutierte Desaturase-Gen aus einer *Brassica napus*-Pflanze stammt.

4. Nucleinsäurefragment nach Anspruch 1, wobei das Gen die D-Form eines mikrosomalen Genes von *Brassicaceae* ist.

5. Pflanze der Familie *Brassicaceae* oder *Helianthus,* mit Ausnahme von *Brassica napus,* wobei die Pflanze eine vollständige Kodierungssequenz für ein Delta-12-Fettsäure-Desaturase-Gen enthält, wobei die Kodierungssequenz zumindest eine Mutation in einem Bereich aufweist, der für ein His-Glu-Cys-Gly-His-Aminosäuremotiv kodiert, wobei die zumindest eine Mutation in dem Motiv ein Codon aufweist, das für Lys kodiert, anstelle des Codons, das für Glu kodiert, und wobei die Mutation in den Samen der Pflanze eine veränderte Fettsäurezusammensetzung verursacht.

6. Pflanze nach Anspruch 5, wobei das Gen aus einer Brassica napus-Pflanze stammt .

7. Pflanze nach Anspruch 5, wobei die Pflanze eine *Brassica* rapa-Pflanze ist.

8. Isoliertes Nucleinsäurefragment, das eine vollständige Kodierungssequenz eines Delta-15-Fettsäure-Desaturase-Genes von *Brassicaceae* aufweist, wobei die Kodierungssequenz zumindest eine Mutation in einem Bereich des Desaturase-Genes aufweist, der für ein His-Asp-Cys-Gly-His-Aminosäuremotiv kodiert, wobei die zumindest eine Mutation in dem Motiv ein Codon aufweist, das für ein Lys kodiert, anstelle des Codons, das für Asp kodiert, und wobei die zumindest eine Mutation das Produkt des Desaturase-Genes nicht-funktionell macht.

9. Nucleinsäurefragment nach Anspruch 8, wobei das mutierte Desaturase-Gen aus einer *Brassica* napus-Pflanze stammt.

10. Brassicaceae-Pflanze, die eine vollständige Kodierungssequenz eines Delta-15-Fettsäure-Desaturase-Genes enthält, das zumindest eine Mutation in einem Bereich aufweist, der für ein His-Asp-Cys-Gly-His-Aminosäuremotiv kodiert, wobei die zumindest eine Mutation in dem Motiv ein Codon aufweist, das für Lys kodiert, anstelle des Codons, das für Asp kodiert, und wobei die Mutation in den Samen der Pflanze eine veränderte Fettsäurezusammensetzung verursacht.

11. Pflanze nach Anspruch 10, wobei das mutierte Desaturase-Gen aus einer *Brassica napus*-Pflanze stammt.

12. Pflanze nach Anspruch 10, wobei die Pflanze eine *Brassica napus*-Pflanze ist.

13. Brassicaceae-Pflanze, die Folgendes aufweist, nämlich:
a) eine vollständige Kodierungssequenz eines Delta-12-Fettsäure-Desaturase-Genes, wobei die Kodierungssequenz zumindest eine Mutation in einem Bereich aufweist, der für ein His-Glu-Cys-Gly-His-Aminosäuremotiv kodiert, wobei die zumindest eine Mutation in dem Motiv ein Codon aufweist, das für Lys kodiert, anstelle des Codons, das für Glu kodiert;
b) eine vollständige Kodierungssequenz eines Delta-15-Fettsäure-Desaturase-Genes, das zumindest eine Mutation aufweist, wobei die zumindest eine Delta-15-Genmutation in einem Bereich für ein His-Xaa-Xaa-Xaa-His-Aminosäuremotiv kodiert, wobei die Delta-12-Genmutation und die Delta-15-Genmutation in den Samen der Pflanze eine veränderte Fettsäurezusammensetzung verursacht.

14. Verfahren zur Herstellung einer Pflanzenlinie von *Brassicaceae* oder *Helianthus,* das folgende Schritte aufweist, nämlich:
a) Induzieren der Mutagenese in Zellen einer Ausgangsvarietät von Spezies von *Brassicaceae* oder *Helianthus;*
b) Erhalten von einer oder mehreren Vorläuferpflanzen aus den Zellen;
c) Identifizieren von zumindest einer der Vorläuferpflanzen, die ein Delta-12-Fettsäure-Desaturase-Gen enthält, das zumindest eine Mutation in einem Bereich aufweist, der für ein His-Glu-Cys-Gly-His-Aminosäuremotiv kodiert, wobei die zumindest eine Mutation in dem Motiv ein Codon aufweist, das für Lys kodiert, anstelle des Codons, das für Glu kodiert, und wobei die zumindest eine Mutation das Produkt des Delta-12-Desaturase-Genes nicht-funktionell macht; und
d) Produzieren der Pflanzenlinie aus der zumindest einen Vorläuferpflanze durch Selbst- oder Fremdbestäubung, wobei die Pflanzenlinie die zumindest eine Delta-12-Genmutation aufweist.

15. Verfahren nach Anspruch 14, wobei die Pflanzenlinie Samen produziert, die ein Öl erbringen, das einen stabilisierten Linolsäuregehalt von etwa 2 % bis etwa 12 % aufweist.

16. Verfahren nach Anspruch 14, das folgende weitere Schritte aufweist, nämlich:
e) Induzieren der Mutagenese in Zellen der Pflanzenlinie;
f) Erhalten von einer oder mehreren Vorläuferpflanzen aus den Zellen der Pflanzenlinie;
g) Identifizieren von zumindest einer der Vorläuferpflanzen der Pflanzenlinie, die ein Delta-15-Fettsäure-Desaturase-Gen enthält, das zumindest eine Delta-15-Genmutation in einem Bereich aufweist, der für ein His-Xaa-Xaa-Xaa-His-Aminosäuremotiv kodiert, wobei die Delta-15-Genmutation das Produkt der Delta-15-Desaturase nicht-funktionell macht;
h) Produzieren von einer zweiten Pflanzenlinie aus der zumindest einen Vorläuferpflanze der Pflanzenlinie durch Selbst- oder Fremdbestäubung, wobei die zweite Pflanzenlinie die zumindest eine Delta-12-Genmutation und die zumindest eine Delta-15-Genmutation aufweist.

17. Verfahren nach Anspruch 14, wobei die Ausgangsvarietät eine Varietät von *Brassica napus* ist.

18. Verfahren nach Anspruch 17, wobei die Mutation in einer ersten Form der Delta-12-Fettsäure-Desaturase liegt.

19. Verfahren nach Anspruch 18, das den weiteren Schritt des Kreuzens einer Pflanze der Pflanzenlinie mit einer solchen Pflanze aufweist, die eine Mutation in einer zweiten Form der Delta-12-Fettsäure-Desaturase aufweist.

20. Verfahren nach Anspruch 19, wobei die zweite Mutation in einem anderen Bereich als in dem Bereich liegt, der für ein His-Xaa-Xaa-Xaa-His-Aminosäuremotiv kodiert.

21. Verfahren nach Anspruch 17, das folgende weitere Schritte aufweist, nämlich:
e) Induzieren der Mutagenese in Zellen der Pflanzenlinie;
f) Erhalten von einer oder mehreren Vorläuferpflanzen aus den Zellen der Pflanzenlinie;
g) Identifizieren von zumindest einer der Vorläuferpflanzen der Pflanzenlinie, die ein zweites Delta-12-Fettsäure-Desaturase-Gen enthält, das zumindest eine Mutation aufweist, wobei die zweite Genmutation in einem anderen Bereich als in dem Bereich liegt, der für ein His-Xaa-Xaa-Xaa-His-Aminosäuremotiv kodiert; und
h) Produzieren von einer zweiten Pflanzenlinie aus der zumindest einen Vorläuferpflanze der Pflanzenlinie durch Selbst- oder Fremdbestäubung, wobei die zweite Pflanzenlinie die erste Delta-12-Genmutation und die zweite Delta-12-Genmutation aufweist.

22. Verfahren zur Herstellung einer Pflanzenlinie von *Brassi*caceae, das folgende Schritte aufweist, nämlich:
a) Induzieren der Mutagenese in Zellen einer Ausgangsvarietät einer Spezies von *Brassicaceae*;
b) Erhalten von einer oder mehreren Vorläuferpflanzen aus den Zellen;
c) Identifizieren von zumindest einer der Vorläuferpflanzen, die ein Delta-15-Fettsäure-Desaturase-Gen enthält, das zumindest eine Mutation in einem Bereich aufweist, der für ein His-Asp-Cys-Gly-His-Aminosäuremotiv kodiert; wobei die zumindest eine Mutation in dem Motiv ein Codon aufweist, das für Lys kodiert, anstelle eines Codons, das für Asp kodiert, und wobei die zumindest eine Mutation das Produkt des Delta-15-Desaturase-Genes nicht-funktionell macht; und
d) Produzieren der Pflanzenlinie aus der zumindest einen Vorläuferzelle durch Selbst- oder Fremdbestäubung, wobei die Pflanzenlinie die zumindest eine Delta-15-Genmutation aufweist.

23. Verfahren nach Anspruch 21 oder 22, wobei der Identifizierungsschritt eine Technik aufweist, die ausgewählt ist aus der Gruppe bestehend aus PCR, 3SR und direkte Polynucleotidsequenzierung.

## Revendications

1. Fragment isolé d'acide nucléique comprenant une séquence complète de codage d'un gène de désaturase d'acide gras delta-12 de *Brassicaceae* ou *d'Helianthus,* la séquence de codage ayant au moins une mutation dans une région du gène de désaturase codant un motif d'acide aminé His-Glu-Cys-Gly-His, la au moins une mutation du motif comprenant un codon codant Lys au lieu du codon codant Glu et ladite au moins une mutation rendant non fonctionnel le produit du gène de désaturase.

2. Fragment d'acide nucléique suivant la revendication 1, dans lequel le gène de désaturase mutant code un produit de gène microsomique.

3. Fragment d'acide nucléique suivant la revendication 1, dans lequel le gène de désaturase mutant provient d'une plante *Brassica napus.*

4. Fragment d'acide nucléique suivant la revendication 1, dans lequel le gène est la forme D d'un gène microsomique de *Brassicaceae.*

5. Plante des familles *Brassicaceae* ou *Helianthus* autre que *Brassica napus,* la plante contenant une séquence complète de codage d'un gène de désaturase d'acide gras delta-12, la séquence de codage ayant au moins une mutation dans une région codant un motif d'acide aminé His-Glu-Cys-Gly-His, ladite au moins une mutation du motif comprenant un codon codant Lys au lieu du codon codant Glu et ladite mutation conférant une composition d'acide gras modifiée aux graines de la plante.

6. Plante suivant la revendication 5, dans laquelle le gène provient d'une plante de *Brassica napus.*

7. Plante suivant la revendication 5, dans laquelle la plante est une plante de *Brassica rapa.*

8. Fragment isolé d'acide nucléique comprenant une séquence complète de codage d'un gène de désaturase d'acide gras delta-15 de *Brassicaceae,* la séquence de codage ayant au moins une mutation dans une région du gène de désaturase codant un motif d'acide aminé His-Asp-Cys-Gly-His, la au moins une mutation du motif comprenant un codon codant Lys au lieu du codon codant Asp et ladite au moins une mutation rendant non fonctionnel le produit du gène de désaturase.

9. Fragment d'acide nucléique suivant la revendication 8, dans lequel le gène de désaturase mutant provient d'une plante *Brassica napus.*

10. Plante de Brassicaceae contenant une séquence complète de codage d'un gène de désaturase d'acide gras delta-15 ayant au moins une mutation dans une région codant un motif d'acide aminé His-Asp-Cys-Gly-His, ladite au moins une mutation du motif comprenant un codon codant Lys au lieu du codon codant Asp et ladite mutation conférant une composition d'acide gras modifiée aux graines de la plante.

11. Plante suivant la revendication 10, dans lequel le gène de désaturase mutant provient d'une plante *Brassica napus.*

12. Plante suivant la revendication 10, dans laquelle la plante est une plante de *Brassica napus*.

13. Plante de *Brassicaceae* contenant :
a) une séquence complète de codage d'un gène de désaturase d'acide gras delta-12, la séquence de codage ayant au moins une mutation dans une région du gène de désaturase codant un motif d'acide aminé His-Glu-Cys-Gly-His, la au moins une mutation du motif comprenant un codon codant Lys au lieu du codon codant Glu ;
b) une séquence complète de codage d'un gène de désaturase d'acide gras delta-15 ayant au moins une mutation, ladite au moins une mutation du gène delta-15 dans une région codant un motif d'acide aminé His-Xaa-Xaa-Xaa-His, la mutation du gène de delta-12 et la mutation du gène de delta-15 conférant une composition modifiée d'acide gras aux graines de la plante.

14. Procédé de production d'une ligne végétale *Brassicaceae* ou *Helianthus* comprenant les stades de :
a) on induit une mutagenèse dans des cellules d'une variété de départ d'une espèce *Brassicaceae* ou *Helianthus ;*
b) on obtient une ou plusieurs plantes de descendance à partir des cellules ;
c) on identifie au moins l'une des plantes de descendance qui contient un gène de désaturase d'acide gras delta-12 ayant au moins une mutation dans une région du gène de désaturase codant un motif d'acide aminé His-Glu-Cys-Gly-His, la au moins une mutation du motif comprenant un codon codant Lys au lieu du codon codant Glu, et ladite au moins une mutation rendant non fonctionnel le produit du gène de désaturase delta-12 ; et
d) on produit la lignée végétale à partir de la au moins une plante de descendance par autopollinisation ou par pollinisation croisée, la lignée végétale ayant ladite au moins une mutation de gène delta-12.

15. Procédé suivant la revendication 14, dans lequel la lignée végétale produit des graines donnant une huile ayant une teneur stabilisée en acide linoléique comprise entre environ 2 % et environ 12 %.

16. Procédé suivant la revendication 14, comprenant en outre les stades de :
e) on induit une mutagenèse dans des cellules de la lignée végétale ;
f) on obtient une ou plusieurs plantes de descendance à partir des cellules de la lignée végétale ;
g) on identifie au moins l'une des plantes de descendance de la lignée végétale qui contient un gène de désaturase d'acide gras delta-15 ayant au moins une mutation de gène delta-15 dans une région codant un motif d'acide aminé His-Xaa-Xaa-Xaa-His, la mutation du gène de delta-15 rendant non fonctionnel le produit de désaturase delta-15 ;
h) on produit une deuxième lignée végétale à partir de la au moins une plante de descendance de la lignée végétale par autopollinisation ou par pollinisation croisée, la deuxième lignée végétale ayant ladite au moins une mutation de gène delta-12 et ladite au moins une mutation de gène delta-15.

17. Procédé suivant la revendication 14, dans lequel la variété de départ est une variété de *Brassica napus.*

18. Procédé suivant la revendication 17, dans lequel la mutation est sous une première forme de désaturase d'acide gras delta-12.

19. Procédé suivant la revendication 18, comprenant en outre le stade de croisement d'une plante de la lignée végétale avec une plante ayant une mutation suivant une deuxième forme de désaturase d'acide gras delta-12.

20. Procédé suivant la revendication 19, dans lequel la deuxième mutation est dans une région autre qu'une région codant un motif d'acide aminé His-Xaa-Xaa-Xaa-His.

21. Procédé suivant la revendication 17, comprenant en outre les stades de :
e) on induit une mutagenèse dans des cellules de la lignée végétale ;
f) on obtient une ou plusieurs plantes de descendance à partir des cellules de la lignée végétale ;
g) on identifie au moins l'une des plantes de descendance de la lignée cellulaire qui contient un deuxième gène de désaturase d'acide gras delta-12 ayant au moins une mutation, la deuxième mutation de gène étant dans une région autre qu'une région codant un motif d'acide aminé His-Xaa-Xaa-Xaa-His ; et
h) on produit une deuxième lignée végétale à partir de ladite au moins une plante de descendance de la lignée végétale par autopollinisation ou par pollinisation croisée, la deuxième lignée végétale ayant ladite première mutation de gène delta-12 et ladite deuxième mutation de gène delta-12.

22. Procédé de production d'une lignée cellulaire de *Brassicaceae* comprenant les stades de :
a) on induit une mutagenèse dans des cellules d'une variété de départ d'une espèce *Brassicaceae ;*
b) on obtient une ou plusieurs plantes de descendance à partir des cellules ;
c) on identifie au moins l'une des plantes de descendance qui contient un gène de désaturase d'acide gras delta-15 ayant au moins une mutation dans une région du gène de désaturase codant un motif d'acide aminé His-Asp-Cys-Gly-His, la au moins une mutation du motif comprenant un codon codant Lys au lieu du codon codant Asp, et ladite au moins une mutation rendant non fonctionnel le produit du gène de désaturase delta-15 ; et
d) on produit la lignée végétale à partir de la au moins une plante de descendance par autopollinisation ou par pollinisation croisée, la lignée végétale ayant ladite au moins une mutation de gène delta-15.

23. Procédé suivant la revendication 21 ou la revendication 22, dans lequel le stade d'identification comprend une technique choisie dans le groupe consistant en PCR, 3SR et le séquençage direct de polynucléotides.
